# EUROPEAN PATENT SPECIFICATION

(11) **EP 1 289 564 B1**
(45) Date of publication and mention of the grant of the patent: **18.07.2018**
(21) Application number: 01941935.7
(22) Date of filing: 05.06.2001
(51) Int. Cl.: A61K 47/66

(54) **T CELL RECEPTOR FUSIONS AND CONJUGATES AND METHODS OF USE THEREOF**
T-ZELL-REZEPTOR FUSIONEN UND KONJUGATE UND VERFAHREN ZU DEREN VERWENDUNG
FUSIONS ET CONJUGUES DE RECEPTEURS DE LYMPHOCYTES T ET PROCEDES D'UTILISATION CORRESPONDANTS

(30) Priority: 05.06.2000 US 209536 P
(43) Date of publication of application: 12.03.2003
(60) Divisional application: 10182289.8
(73) Proprietor: Altor BioScience Corporation, Miramar, Florida 33025 (US)
(72) Inventor: WEIDANZ, Jon, A., Amarillo ,Texas 79124 (US); CARD, Kimberlyn, F., Pembroke Pines, FL 33025 (US); WONG, Hing, C., Weston, FL 33322 (US)
(74) Representative: Hiebl, Inge Elisabeth
(86) International application number: PCT/US2001/018145
(87) International publication number: WO 2001/093913

(56) References cited:
- EP-A- 0 659 438
- WO-A-00/23087
- WO-A-96/13593
- WO-A-98/39482
- WO-A-99/18129
- WO-A2-94/12648
- US-A- 5 723 309
- DATABASE BIOSIS [Online] BIOSCIENCES INFORMATION SERVICE, PHILADELPHIA, PA, US; 8 March 2001 (2001-03-08) CARD KIMBERLYN F ET AL: "Novel bifunctional molecules for cancer immunotherapy." Database accession no. PREV200100264679 XP002213808 & FASEB JOURNAL, vol. 15, no. 5, 8 March 2001 (2001-03-08), page A1200 Annual Meeting of the Federation of American Societies for Experimental Biology on Experimental Biology 2001;Orlando, Florida, USA; March 31-April 04, 2001 ISSN: 0892-6638

### SEQUENCE LISTING

<110> SUNOL MOLECULAR CORPORATION
<120> T CELL RECEPTOR FUSIONS AND CONJUGATES AND METHODS OF USE THEREOF
<130> 12878EP
<140> 01 941 935.7
   <141> 2001-06-05
<150> 60/209,536
   <151> 2000-06-05
<160> 3
<170> PatentIn Ver. 2.1
<210> 1
   <211> 10
   <212> PRT
   <213> Artificial Sequence
<220>
   <223> Description of Artificial Sequence:Peptide linker
<400> 1
   Ala Ser Gly Gly Gly Gly Ser Gly Gly Gly
    1               5                  10
<210> 2
   <211> 9
   <212> PRT
   <213> Artificial Sequence
<220>
   <223> Description of Artificial Sequence:Protein tag
<400> 2
   Glu Glu Glu Glu Tyr Met Pro Met Glu
    1               5
<210> 3
   <211> 33
   <212> DNA
   <213> Artificial Sequence
<220>
   <223> Description of Artificial Sequence:Primer
<400> 3
   tttcgtacgt cttgtcccag tcagtgacgc agc 33

## Description

### FIELD OF INVENTION

The present invention relates to soluble T cell receptor complexes and more particularly to soluble T cell receptor fusion complexes and soluble T cell receptor conjugate complexes, as well as methods for making and using such molecules. The provided molecules are useful for a variety of therapeutic applications as well as diagnostic purposes.

### BACKGROUND OF THE INVENTION

Traditional approaches to the treatment of diseases such as cancers, autoimmune, and infective (including viral, bacterial, parasitic and fungal) diseases, have included surgery, radiation chemotherapy, antibiotics or combination therapies. However, such therapies have not proven effective against a majority of these indications. Development of alternate remedies for preventing and/or treating human diseases is crucial. In recent years immunotherapy and gene therapy approaches utilizing antibodies and T-lymphocytes have emerged as new and promising methods for treating human disease.

One such approach to treatment has included use of antibodies for targeting of therapeutic or diagnostic agents to particular targets. Numerous groups have made developments revolving around the use of antibodies as a targeting agent. Such developments have included construction of antibody fusion proteins and antibody conjugate molecules linking antibodies to various effector molecules, including radioactive molecules, chemotherapeutics agents, toxins, and additional bioactive proteins. Therapeutics or diagnostics developed using such molecules are designed to cause a particular effect which is targeted by the linked antibody.

Just as antibodies have been developed as therapeutics, additional primary effectors of the immune system, T cell receptors (TCR), have unique advantages as a platform for developing therapeutics. While antibodies are limited to recognition of pathogens in the blood and extracellular spaces or to protein targets on the cell surface, T cell receptors can recognize antigens displayed with MHC molecules on the surfaces of cells (including antigens derived from intracellular proteins). Depending on the subtype of T cells that recognize displayed antigen and become activated, T cell receptors and T cells harboring T cell receptors can participate in controlling various immune responses. For instance, T cells are involved in regulation of the humoral immune response through induction of differentiation of B cells into antibody producing cells. In addition, activated T cells act to initiate cell-mediated immune responses. Thus, T cell receptors can recognize additional targets not available to antibodies.

A T-cell response is modulated by antigen binding to a T-cell receptor (TCR). One type of TCR is a membrane bound heterodimer consisting of an α and β chain resembling an immunoglobin variable (V) and constant (C) region. The TCR α chain includes a covalently linked V-α and C-α chain, whereas the β chain includes a V-β chain covalently linked to a C-β chain. The V-α and V-β chains form a pocket or cleft that can bind a superantigen or antigen in the context of a major histocompatibility complex (MHC) (known in humans as an HLA complex). See generally Davis Ann. Rev. of Immunology 3: 537 (1985); Fundamental Immunology 3rd Ed., W. Paul Ed. Rsen Press LTD. New York (1993).

WO 2000/023087 discloses polyspecific binding molecules and particularly single-chain polyspecific binding molecules that include at least one single-chain T-cell receptor (sc-TCR) covalently linked through a peptide linker sequence to at least one single-chain antibody (sc-Ab). Further disclosed are methods and compositions for testing and using the molecules.

WO 1999/018129 features fully soluble and functional single-chain T-cell receptor proteins. In one aspect, WO 1999/018129 features soluble fusion proteins comprising an immunoglobin light chain constant region or fragment thereof covalently linked to a single-chain T-cell receptor, wherein the single-chain T-cell receptor comprises a V-$g(a) chain covalently linked to a V-$g(b) chain by a peptide linker sequence. The single-chain T-cell receptor proteins have a variety of uses including use in screens to detect cells expressly desired MHC-peptide complexes.

WO 1994/012648 relates to a method for producing soluble T-receptors, wherein DNA sequences coding for each of the T-receptor subunits deleted from the T-receptor transmembrane portion are co-transfected in a host cell. The resulting products and their diagnostic and therapeutical uses are also disclosed.

The TCR is believed to play an important role in the development and function of the immune system. For example, the TCR has been reported to mediate cell killing, increase B cell proliferation, and impact the development and severity of various disorders including cancer, allergies, viral infections and autoimmune disorders.

It thus would be desirable to provide novel targeting agents based on T cell receptors, as well as methods for producing and using such agents for therapeutic and diagnostic settings. It would be particularly desirable to provide such molecules that would have certain advantages in comparison to prior art complexes based an antibody targeting.

### SUMMARY OF THE INVENTION

We have now created several different modified TCR complexes that have potential therapeutic utility. These modified TCRs can be used to guide, target or direct localized toxic agents to specific sites to intervene in a disease process. For example, a TCR, which specifically recognizes a peptide derived from a cancer associated protein that is displayed by an MHC molecule, can be fused or conjugated to a biologically active molecule and thereby guide that molecule to the cancer cell to effect a desirable therapeutic outcome.

The TCRs of the invention are modified in ways that link the TCR to the biologically active molecule. This invention teaches the use of genetic fusions and chemical conjugation as methods for effecting such linkage. The TCR to which the biologically active molecule is attached is a soluble, single-chain TCR. The biologically active molecule is an interleukin-2 (IL-2).

In some instances, the soluble sc-TCR proteins will include one or more fused effectors or tags in addition to the above biologically active molecule. For example, in some cases the tags can be used to help purify the TCR protein fusion complex from naturally-occurring cell components which typically accompany the fusion protein. In other cases, the protein tag can be used to introduce a pre-determined chemical or proteolytic cleavage site into the soluble protein. Particularly, contemplated is introduction of a segment encoding a tag into a DNA vector, e.g., between sequence encoding the fusion complex and the effector molecule chain or suitable fragment so that the TCR molecule can be cleaved (i.e. separated) from the effector chain or fragment if desired.

T cell receptor molecules for use in the invention are soluble single chain T cell receptors.

The TCR fusion complex is covalently linked to a cytokine being IL-2.

In another preferred aspect of the invention, a TCR fusion complex is additionally covalently linked to a radioactive materials, such as I¹²⁵ for example.

Still another preferred aspect of the invention includes, a TCR fusion complex is additionally covalently linked to a detectable labels, such as fluorescent, radioactive or electron transfer agents, for example.

Specifically provided are soluble TCR fusion proteins and TCR conjugate complexes that additionally include an effector that is a cell toxin or a detectably-labelled atom or compound suitable for diagnostic, imaging, or therapeutic studies. The TCR fusion complexes and TCR conjugate complexes can be used in a variety of applications including detection and/or imaging cells or tissue *in vivo,* as well as therapeutic uses such as damaging or killing cells *in vitro* or *in vivo.* In general, targeted cells or tissue will include one or more ligands capable of selectively binding the TCR. Exemplary cells include tumor cells such as melanoma and virally-infected cells (e.g., cells infected with a primate DNA or RNA virus such as cytomegalovirus or the AIDS virus, respectively).

Other aspects and embodiments of the invention are discussed below.

### BRIEF DESCRIPTION OF THE DRAWINGS

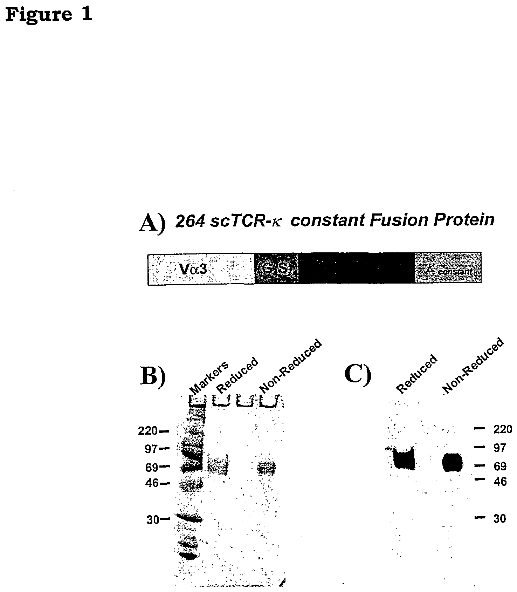

Figure 1 is a construct design and expression of soluble 264 single-chain (sc) T cell receptor-kappa constant chain fusion protein (TCR-κ).
   A) A schematic representing the 264 TCR constructed as a three-domain scTCR covalently linked to the kappa constant chain region.
   B) Coomassie blue stain of a protein gel containing purified 264 scTCR-κ fusion protein run under reduced and non-reduced conditions.
   C) Immuno-blot of purified 264 scTCR-κ fusion protein probed with an anti-kappa-horseradish peroxidase (HRP)-labeled conjugate.

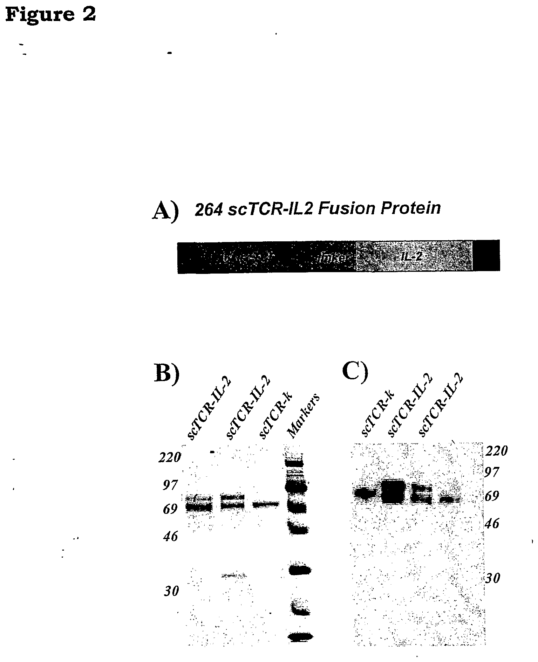

Figure 2 represents the construct design and expression of a soluble 264 scTCR-IL-2 fusion protein.
   A) A schematic showing the 264 scTCR gene covalently linked to the IL-2 gene with the EE peptide tag included to facilitate detection of the molecule.
   B) Coomassie blue stain of a protein gel containing purified 264 scTCR-IL-2 and 264 scTCR-κ fusion proteins.
   C) Immunoblot analysis of the purified 264 scTCR-IL-2 fusion protein probed with an anti-EE tag mAb and a goat antimouse-HRP conjugate.
Figure 3 is demonstrative results of IL-2 activity in a bioassay.
Figure 4 is demonstrative results of an antigen presenting cell stained with the 264 scTCR-IL2 fusion protein.
Figure 5 is demonstrative results of a cell conjugation assay.

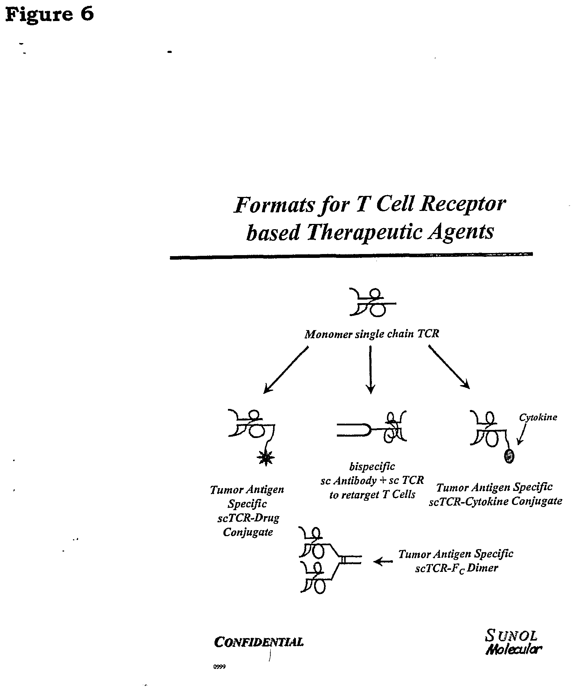

Figure 6 a schematic for formats for T cell receptor based therapeutic agents.

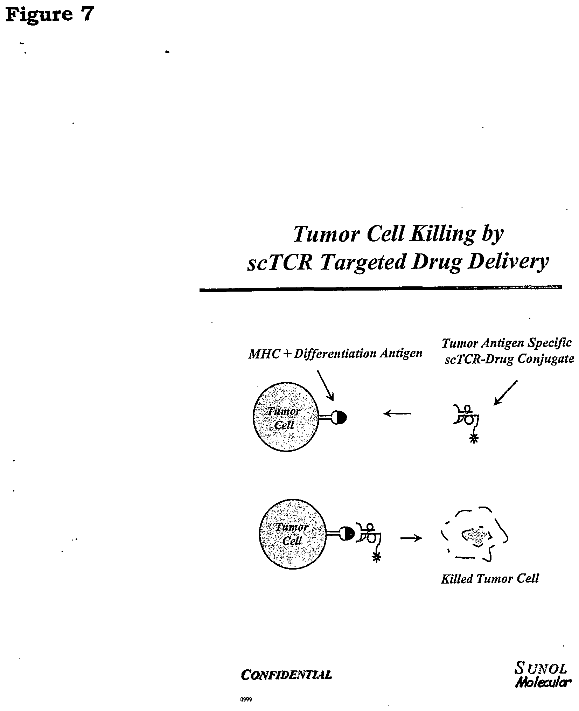

Figure 7 is a schematic of tumor cell killing mediated by scTCR targeted drug delivery.

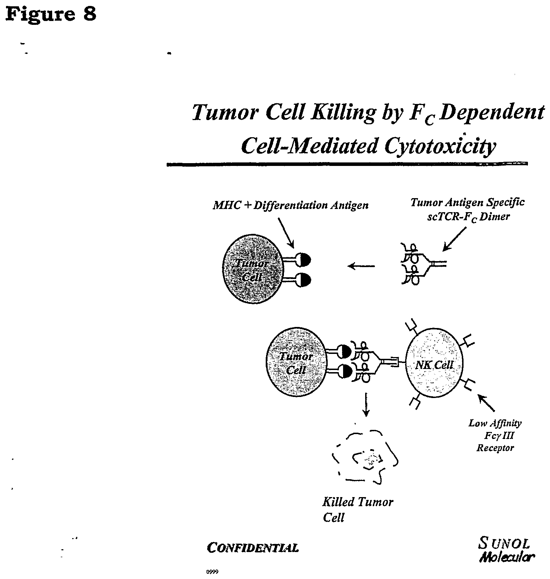

Figure 8 is a schematic of tumor cell killing mediated by Fc dependent cell-mediated cytotoxicity.
Figure 9 is a schematic illustration of the pNAG2 vector.
Figure 10 is a schematic drawing showing the pSUN27 vector. Figure 11 is a drawing showing preferred bispecific hybrid molecules pBISP/D011.10 and pBISP/149.
Figure 12A is a schematic drawing showing a method for making a chimeric bispecific antibody molecule. The method uses a hybridoma-expressing cell (145-2C11 hybridoma) to produce antibody chains (heavy lines) that combine with an sc-TCR/Ig fusion molecule (light chain) inside the cell. A preferred structure for the sc-TCR/Ig molecule is illustrated in Figure 12B.
Figure 13 is a schematic drawing showing the vector pSUN7 vector.

### DETAILED DESCRIPTION OF THE INVENTION

In an attempt to improve upon the performance of antibody-based molecules, we have developed antigen-specific therapeutics based on use of T cell receptors (TCRs). TCR-based reagents would have several advantages over antibody molecules. First, antibody-based therapies are often associated with lower than expected killing efficiency of tumor cells due to shedding of tumor antigens. Although there are reports of MHC shedding, the levels of specific MHC/tumor peptide in circulation are much lower than free circulating tumor antigen. Second, antibody molecules fail to recognize many potential tumor antigens because they are not exposed on the surface of the cells or not accessible to the antibody molecule. Many potential tumor specific proteins are intracellular but are normally processed within the cell into peptides which are then presented in the context of either MHC class I or MHC class II molecules on the surface of the tumor cell. Unlike TCRs, antibodies do not generally recognize these processed antigens occupying the binding clefts of MHC molecules. Third, many of the antigens recognized by antibodies are heterogeneic by nature, which limits the effectiveness of an antibody to a single tumor histology. In contrast, many T cell epitopes are common to a broad range of tumors originating from several distinct tissues.

As summarized above, we have now created TCR fusion and conjugate complexes, and expression vectors that encode such complexes, that comprise a soluble single-chain TCR molecule covalently linked to an interleukin-2 (IL-2), and methods for production and use of such fusion and conjugate complexes and expression vectors and conjugate complexes.

A T cell recognizes antigen presented on the surfaces of cells by means of the T cell receptors expressed on their cell surface. TCRs are disulfide linked heterodimers, most consisting of α and β chain glycoproteins. T cells use mechanisms to generate diversity in their receptor molecules similar to those mechanisms for generating antibody diversity operating in B cells (Janeway and Travers; Immunobiology 1997). Similar to the immunoglobulin genes, TCR genes are composed of segments that rearrange during development of T cells. TCR polypeptides consist of amino terminal variable and carboxy terminal constant regions. While the carboxy terminal region functions as a trans-membrane anchor and participates in intracellular signaling when the receptor is occupied, the variable region is responsible for recognition of antigens. The TCR α chain contains variable regions encoded by V and D segments only, while the β chain contains additional joining (J) segments. The rearrangement of these segments and the mutation and maturation of the variable regions results in a diverse repertoire of TCRs capable of recognizing an incredibly large number of different antigens displayed in the context of different TCR molecules.

Technology has been developed previously to produce highly specific T cell receptors (TCR) which recognize particular antigen. For example, the pending U. S. patent applications U.S.S.N. 08/813,781 and U.S.S.N.
09/422,375; and International publications PCT/US98/04274 and PCT/US99/24645, and references discussed therein disclose methods of preparing and using specific TCRs. Additionally, particular specific TCRs have been produced by recombinant methods as soluble, single-chain TCRs (scTCR). Methods for production and use of scTCRs have been disclosed and are described in pending U.S. patent application 08/943,086, and International application PCT/US98/20263. Such TCRs and scTCRs can be altered so as to create fusions or conjugates to render the resulting TCRs and scTCRs useful as therapeutics. The TCR complexes of the invention can be generated by genetically fusing the recombinantly produced TCR or scTCR coding region to genes encoding biologically active protein being an interleukin-2 (IL-2) to produce TCR fusion complexes. Alternatively, a TCR or scTCRs can also be chemically conjugated with biologically active molecule being an interleukin-2 (IL-2) to produce TCR conjugate complexes.

By the term "fusion molecule" as it is used herein is meant a soluble single-chain TCR molecule and an effector molecule that is an interleukin-2 (IL-2), covalently linked (i.e. fused) by recombinant, chemical or other suitable method. If desired, the fusion molecule can be fused at one or several sites through a peptide linker sequence. Alternatively, the peptide linker may be used to assist in construction of the fusion molecule. Specifically preferred fusion molecules are fusion proteins.

A "polypeptide" refers to any polymer preferably consisting essentially of any of the 20 natural amino acids regardless of its size. Although the term "protein" is often used in reference to relatively large proteins, and "peptide" is often used in reference to small polypeptides, use of these terms in the field often overlaps. The term "polypeptide" refers generally to proteins, polypeptides, and peptides unless otherwise noted. Peptides useful in accordance with the present invention in general will be generally between about 0.1 to 100 KD or greater up to about 1000 KD, preferably between about 0.1, 0.2, 0.5, 1, 2, 5, 10, 20 ,30 and 50 KD as judged by standard molecule sizing techniques such as centrifugation or SDS-polyacrylamide gel electropheresis.

By the term "conjugate molecule" as it is used herein is meant a soluble single-chain TCR molecule and an effector molecule being an interleukin-2 (IL-2), covalently linked (i.e. fused) by chemical or other suitable method. If desired, the conjugate molecule can be fused at one or several sites through a peptide linker sequence or a carrier molecule. Alternatively, the peptide linker or carrier may be used to assist in construction of the conjugate molecule. Specifically preferred conjugate molecules additionally comprise toxins or detectable labels.

TCR fusion and TCR conjugate complexes of the invention comprise a biologically active or effector molecule (terms to be used herein interchangeably) covalently linked to the TCR molecule being an interleukin-2 (IL-2). Desired effects of the invention include, for example, either to induce cell proliferation or cell death, initiate an immune response or to act as a detection molecule for diagnostic purposes as determined by the assays disclosed below, including an assay that includes sequential steps of culturing cells to proliferate same, and contacting the cells with a TCR fusion complex of the invention and then evaluating whether the TCR fusion complex inhibits further development of the cells.

The present invention is directed to a soluble single-chain T cell receptor fusion complex comprising a single-chain T cell receptor polypeptide and a biologically active polypeptide connected by a peptide linker, wherein the biologically active polypeptide is an interleukin-2 (IL-2).

In one embodiment, said single-chain T cell receptor fusion complex further comprises a plurality of biologically active molecules, wherein the biologically active molecules are selected from the group consisting of cytotoxic molecules, toxins, chemotherapeutic agents, anti-cancer drugs, detectable labels, fluorescent compounds, electron transfer agents, enzymes, and radioactive compounds, wherein the biologically active molecules are covalently linked to the soluble single-chain T cell receptor conjugate complex, wherein the resulting conjugate is soluble.
Cytokines of the invention are defined by any factor produced by cells that affect other cells and are responsible for any of a number of multiple effects of cellular immunity. Examples of cytokines include but are not limited to IL2, IL10, IL-4, IL-12 and INF-γ.

Chemokines of the invention, similar to cytokines, are defined as any chemical factor or molecule which when exposed to other cells are responsible for any of a number of multiple effects of cellular immunity. Suitable chemokines may include but are not limited to MIP-1β, IL-8, MCP-1, and Rantes.

Growth factors include any molecules which when exposed to a particular cell induce proliferation and/or differentiation of the affected cell. Growth factors include proteins and chemical molecules, some of which include: human growth factor and stem cell growth factor. Additional growth factors may also be suitable for uses described herein.

Toxins or cytotoxic agents include any substance which has a lethal effect or an inhibitory effect on growth when exposed to cells. More specifically, the effector molecule can be a cell toxin of, e.g., plant or bacterial origin such as, e.g., diphtheria toxin (DT), shiga toxin, abrin, cholera toxin, ricin, saporin, pseudomonas exotoxin (PE), pokeweed antiviral protein, or gelonin. Biologically active fragments of such toxins are well known in the art and include, e.g., DT A chain and ricin A chain. Additionally, the toxin can be an agent active at the cell surface such as, e.g., phospholipase enzymes (e.g., phospholipase C).

Further, the effector molecule can be a chemotherapeutic drug such as, e.g., vindesine, vincristine, vinblastin, methotrexate, adriamycin, bleomycin, or cisplatin.

Additionally, the effector molecule can be a detectably-labelled molecule suitable for diagnostic or imaging studies such as a fluorescent label such as green fluorescent protein, phycoerythrin, cychome, or texas red; or a radionuclide e.g., iodine-131, yttrium-90, rhenium-188 or bismuth-212. See e.g., Moskaug, et al. J. Biol. Chem. 264, 15709 (1989); Pastan, I. et al. Cell 47, 641, 1986; Pastan et al., Recombinant Toxins as Novel Therapeutic Agents, Ann. Rev. Biochem. 61, 331, (1992); *"*Chimeric Toxins" Olsnes and Phil, Pharmac. Ther., 25, 355 (1982); published PCT application no. WO 94/29350; published PCT application no. WO 94/04689; and U.S. Pat. 5,620,939 for disclosure relating to making and using proteins comprising effectors or tags.

A TCR fusion or conjugate complex that includes a covalently linked effector molecule being an interleukin-2 (IL-2) has several important uses. For example, the TCR fusion or conjugate complex can be employed to deliver the effector molecule to certain cells capable of specifically binding the TCR. Accordingly, the TCR fusion or conjugate complex provide means of selectively damaging or killing cells comprising the ligand. Examples of cells or tissue capable of being damaged or killed by the TCR fusion or conjugate complexes include tumors and virally or bacterially infected cells expressing one or more ligands capable of being specifically bound by the TCR. Cells or tissue susceptible to being damaged or killed can be readily assayed by the methods disclosed herein.

A specific example of a TCR fusion complex fused to an effector molecule is as follows: an sc-TCR such as the p264 sc-TCR disclosed below in Examples 5 below can be produced by transfecting mammalian cells with 264 DNA vector illustrated in Fig. 1. The sc-TCR p264 protein fusion complex recognizes a processed peptide fragment from human wild-type p53 tumor suppressor protein presented in the context of human HLA antigen;
HLA-2.1. The sc-TCR p264 and its peptide ligand have been described in Theobald, M.J., et al., PNAS (USA) (1995), 92:11993. The peptide sequence is LLGRNSFEV. Expression of tumor suppressor protein p53, is upregulated on malignant cells. It has been shown that 50% of all tumors expressed increased levels of p53 on the surface (Holliston, M.D., et al., Science (1991), 253:49). Therefore, scTCR molecules specific for this epitope could be labeled with a toxin that could than be delivered to the malignant cells expressing the p53 peptide fragment HLA-2.1 ligand. This target specific immunotherapy could be effective at killing only malignant cells. Methods for measuring cytotoxicity in *vitro* are well-known and include conventional viability assays as described below.

A sc-TCR molecule comprising p149 sc-TCR linked to an effector has other important uses. For example, the sc-TCR molecule can be used to selectively kill human breast cancer cells expressing 264 peptide. *In vitro* studies can be conducted in which the ability of the toxin labeled 264 molecule to kill breast cancer cells is evaluated using a non-radioactive cell cytotoxic assay using a Eu³⁺ release cytotoxicity assay (Bouma, G.J., et al., (1992) Hum. Immunol. 35:85). A sc-TCR molecule comprising a fused effector molecule can be readily tested *in vivo.* For example, in *vitro* studies can be carried out by grafting p264/HLA.A21 expressing breast cancer cells into HLA/A2 transgenic mouse. (Theobald, et al., (1995) *supra*). Toxin labeled scTCR p264 molecules can be injected into mice at pre-determined dosages and the effect on tumor size can be measured to indicate efficacy of the sc-TCR molecules. In addition, extension of life can be used as a second criterion to evaluate the efficiency of the novel anti-tumor therapy.

Other suitable effector or tag molecules are known. For example, one tag is a polypeptide bearing a charge at physiological pH, such as, e.g., 6xHIS. In this instance, the TCR fusion or conjugate complex can be purified by a commercially available metallo-sepharose matrix such as Ni-sepharose which is capable of specifically binding the 6xHIS tag at about pH 6-9. The EE epitope and myc epitope are further examples of suitable protein tags, which epitopes can be specifically bound by one or more commercially available monoclonal antibodies.

In some settings it can be useful to make the TCR fusion or conjugate complexes of the present invention polyvalent, e.g., to increase the valency of the sc-TCR. Briefly stated, the polyvalent TCR protein is made by covalently linking together between one and four proteins (the same or different) by using e.g., standard biotin-streptavidin labeling techniques, or by conjugation to suitable solid supports such as latex beads. Chemically cross-linked proteins (for example cross-linked to dendrimers) are also suitable polyvalent species. For example, the protein can be modified by including sequences encoding amino acid residues with chemically reactive side chains such as Cys or His. Such amino acids with chemically reactive side chains may be positioned in a variety of positions in the fusion protein, preferably distal to the antigen binding region of the TCR. For example, the C-terminus of a C-β chain fragment of a soluble fusion protein can be covalently linked to a protein purification tag or other fused protein which includes such a reactive amino acid(s). Suitable side chains can be included to chemically link two or more fusion proteins to a suitable dendrimer particle to give a multivalent molecule. Dendrimers are synthetic chemical polymers that can have any one of a number of different functional groups of their surface (D. Tomalia, Aldrichimica Acta, 26:91:101 (1993)). Exemplary dendrimers for use in accordance with the present invention include e.g. E9 starburst polyamine dendrimer and E9 combust polyamine dendrimer, which can link cysteine residues.

As used herein, the term "cell" is intended to include any primary cell or immortalized cell line, any group of such cells as taken from, a tissue or an organ. Preferably the cells are of mammalian and particularly of human origin, and can be infected by one or more pathogens. A "host cell" in accord with the invention can be an infected cell or it can be a cell such as *E*. *coli* that can be used to propagate a nucleic acid described herein.

Covalently linking the effector molecule to the TCR peptide in accordance with the invention provides a number of significant advantages. TCR fusion complexes of the invention can be produced that contain a single effector molecule, including such a peptide of known structure. Additionally, a wide variety of effector molecules can be produced in similar DNA vectors. That is, a library of different effector molecules can be linked to the TCR molecule for presentation of infected or diseased cells. Further, for therapeutic applications, rather than administration of an TCR molecule to a subject, a DNA expression vector coding for the TCR molecule linked to the effector peptide can be administered for in vivo expression of the TCR fusion complex. Such an approach avoids costly purification steps typically associated with preparation of recombinant proteins and avoids the complexities of antigen uptake and processing associated with conventional approaches.

As noted, components of the fusion proteins disclosed herein, e.g., biologically active products being interleukin-2 (IL-2) and any peptide linkers, can be organized in nearly any fashion provided that the fusion protein has the function for which it was intended. In particular, each component of the fusion protein can be spaced from another component by at least one suitable peptide linker sequence if desired. Additionally, the fusion proteins may include tags, e.g., to facilitate identification and/or purification of the fusion protein. More specific fusion proteins are in the Examples described below.

TCR fusion complexes of the invention also include a flexible peptide linker sequence interposed between the TCR protein and the biologically active peptide. The linker sequence should allow effective positioning of the biologically active peptide with respect to the TCR molecule binding groove so that the T cell receptor can recognize presenting MHC-peptide complexes and can deliver the biologically active molecules to a desired site. Successful presentation of the effector molecule can modulate the activity of a cell either to induce or to inhibit T-cell proliferation, or to initiate or inhibit an immune response to a particular site, as determined by the assays disclosed below, including the in vitro assays that includes sequential steps of culturing T cells to proliferate same, and contacting the T cells with a TCR fusion complex of the invention and then evaluating whether the TCR fusion complex inhibits further development of the cells.

In general, preparation of the TCR fusion complexes of the invention can be accomplished by procedures disclosed herein and by recognized recombinant DNA techniques involving, e.g., polymerase chain amplification reactions (PCR), preparation of plasmid DNA, cleavage of DNA with restriction enzymes, preparation of oligonucleotides, ligation of DNA, isolation of mRNA, introduction of the DNA into a suitable cell, transformation or transfection of a host, culturing of the host. Additionally, the fusion molecules can be isolated and purified using chaotropic agents and well known electrophoretic, centrifugation and chromatographic methods. See generally, Sambrook et al., Molecular Cloning: A Laboratory Manual (2nd ed. (1989); and Ausubel et al., Current Protocols in Molecular Biology, John Wiley & Sons, New York (1989) for disclosure relating to these methods.

The invention further provides nucleic acid sequences and particularly DNA sequences that encode the present fusion proteins. Preferably, the DNA sequence is carried by a vector suited for extrachromosomal replication such as a phage, virus, plasmid, phagemid, cosmid, YAC, or episome. In particular, a DNA vector that encodes a desired fusion protein can be used to facilitate preparative methods described herein and to obtain significant quantities of the fusion protein. The DNA sequence can be inserted into an appropriate expression vector, i.e., a vector which contains the necessary elements for the transcription and translation of the inserted protein-coding sequence. A variety of host-vector systems may be utilized to express the protein-coding sequence. These include mammalian cell systems infected with virus (e.g., vaccinia virus, adenovirus, etc.); insect cell systems infected with virus (e.g., baculovirus); microorganisms such as yeast containing yeast vectors, or bacteria transformed with bacteriophage DNA, plasmid DNA or cosmid DNA. Depending on the host-vector system utilized, any one of a number of suitable transcription and translation elements may be used. See generally Sambrook et al., *supra* and Ausubel et al. *supra.*

In general, a preferred DNA vector according to the invention comprises a nucleotide sequence linked by phosphodiester bonds comprising, in a 5' to 3' direction a first cloning site for introduction of a first nucleotide sequence encoding a single soluble TCR chain, operatively linked to a sequence encoding an effector molecule, wherein said effector molecule is a cytokine or a fragment thereof, a growth factor or a fragment thereof, a chemokine or a fragment thereof, or GCSF or a fragment thereof.

In most instances, it will be preferred that each of the fusion protein components encoded by the DNA vector be provided in a "cassette" format. By the term "cassette" is meant that each component can be readily substituted for another component by standard recombinant methods. In particular, a DNA vector configured in a cassette format is particularly desirable when the encoded fusion complex is to be used against pathogens that may have or have capacity to develop serotypes.

To make the vector coding for a TCR fusion complex, the sequence coding for the TCR molecule is linked to a sequence coding for the effector peptide by use of suitable ligases. DNA coding for the presenting peptide can be obtained by isolating DNA from natural sources such as from a suitable cell line or by known synthetic methods, e.g. the phosphate triester method. See, e.g., Oligonucleotide Synthesis, IRL Press (M.J. Gait, ed., 1984). Synthetic oligonucleotides also may be prepared using commercially available automated oligonucleotide synthesizers. Once isolated, the gene coding for the TCR molecule can be amplified by the polymerase chain reaction (PCR) or other means known in the art. Suitable PCR primers to amplify the TCR peptide gene may add restriction sites to the PCR product. The PCR product preferably includes splice sites for the effector peptide and leader sequences necessary for proper expression and secretion of the TCR-effector fusion complex. The PCR product also preferably includes a sequence coding for the linker sequence, or a restriction enzyme site for ligation of such a sequence.

The fusion proteins described herein are preferably produced by standard recombinant DNA techniques. For example, once a DNA molecule encoding the TCR protein is isolated, sequence can be ligated to another DNA molecule encoding the effector polypeptide. The nucleotide sequence coding for a TCR molecule may be directly joined to a DNA sequence coding for the effector peptide or, more typically, a DNA sequence coding for the linker sequence as discussed herein may be interposed between the sequence coding for the TCR molecule and the sequence coding for the effector peptide and joined using suitable ligases. The resultant hybrid DNA molecule can be expressed in a suitable host cell to produce the TCR fusion complex. The DNA molecules are ligated to each other in a 5' to 3' orientation such that, after ligation, the translational frame of the encoded polypeptides is not altered (i.e., the DNA molecules are ligated to each other in-frame). The resulting DNA molecules encode an in-frame fusion protein.

Other nucleotide sequences also can be included in the gene construct. For example, a promoter sequence, which controls expression of the sequence coding for the TCR peptide fused to the effector peptide, or a leader sequence, which directs the TCR fusion complex to the cell surface or the culture medium, can be included in the construct or present in the expression vector into which the construct is inserted. An immunoglobulin or CMV promoter is particularly preferred.

The components of the fusion protein can be organized in nearly any order provided each is capable of performing its intended function. For example, in one embodiment, the TCR is situated at the C or N terminal end of the effector molecule.

Preferred effector molecules of the invention will have sizes conducive to the function for which those domains are intended. The effector molecules of the invention can be made and fused to the TCR by a variety of methods including well-known chemical cross-linking methods. See e.g., Means, G.E. and Feeney, R.E. (1974) in Chemical Modification of Proteins, Holden-Day. See also, S.S. Wong (1991) in Chemistry of Protein Conjugation and Cross-Linking, CRC Press. However it is generally preferred to use recombinant manipulations to make the in-frame fusion protein.

As noted, a fusion molecule or a conjugate molecule in accord with the invention can be organized in several ways. In an exemplary configuration, the C-terminus of the TCR is operatively linked to the N-terminus of the effector molecule. That linkage can be achieved by recombinant methods if desired. However, in another configuration, the N-terminus of the TCR is linked to the C-terminus of the effector molecule.

In addition, one or more additional effector molecules can be inserted into the TCR fusion or conjugate complexes as needed.

Preferred fusion and conjugate complexes in accord with the present invention typically include operatively linked in sequence (N to C terminus): 1) a TCR/one or more linker molecules/and a biologically active molecule; 2) TCR/ linker molecule /and a biologically active molecule; and 3) TCR/a first linker molecule /a first biologically active molecule subunit/a second linker molecule /and a second biologically active molecule subunit. In addition, one or more protein tags such as EE, HA, Myc, and polyhistidine, particularly 6Xhis, can be fused to the N-terminus of the TCR chains as desired, e.g., to improve solubility or the facilitate isolation and identification of the TCR fusion and conjugate complexes.

The linker sequence is preferably a nucleotide sequence that codes for a peptide that can effectively position the binding groove of the TCR molecule for recognition of a presenting antigen. As used herein, the phrase "biologically active peptide is effectively positioned linked to a TCR molecule", or other similar phrase, is intended to mean the biologically active peptide linked to a TCR protein is positioned so that the biologically active peptide is capable of interacting with effector cells and modulating the activity of a presenting cell, either to induce cell proliferation, to initiate or inhibit an immune reaction, or to inhibit or inactivate cell development as determined by an assay disclosed below, including the assay that includes sequential steps of culturing cells to proliferate same, and contacting the cells with a TCR fusion complex of the invention and then evaluating whether the TCR fusion complex inhibits further development of the cells.

Preferably the linker sequence comprises from about 7 to 20 amino acids, more preferably from about 8 to 16 amino acids. The linker sequence is preferably flexible so as not hold the biologically active peptide in a single undesired conformation. The linker sequence can be used, e.g., to space the recognition site from the fused molecule. Specifically, the peptide linker sequence can be positioned between the TCR chain and the effector peptide, e.g., to chemically cross-link same and to provide molecular flexibility. The linker is preferably predominantly comprises amino acids with small side chains, such as glycine, alanine and serine, to provide for flexibility. Preferably about 80 or 90 percent or greater of the linker sequence comprises glycine, alanine or serine residues, particularly glycine and serine residues. For a TCR fusion complex that contains a heterodimer TCR, although only soluble single-chain TCRs are encompassed by the present invention, it is contemplated that the linker sequence is suitably linked to the β chain of the TCR molecule, although the linker sequence also could be attached to the α chain of the TCR molecule. Alternatively, linker sequence may be linked to both α and β chains of the TCR molecule. For covalently linking an effector molecule peptide to a TCR β chain molecule, the amino sequence of the linker should be capable of spanning suitable distance from the N-terminal residue of the TCR β chain to the C-terminal residue of the effector molecule peptide. When such a β+peptide chain is expressed along with the α chain, the linked TCR-effector peptide should fold resulting in a functional TCR molecule as generally depicted in Figure 1. One suitable linker sequence is ASGGGGSGGG SEQ ID NO: 1 (i.e., Ala Ser Gly Gly Gly Gly Ser Gly Gly Gly), preferably linked to the first amino acid of the β domain of the TCR. Different linker sequences could be used including any of a number of flexible linker designs that have been used successfully to join antibody variable regions together, see Whitlow, M. et al., (1991) Methods: A Companion to Methods in Enzymology 2:97-105. Suitable linker sequences can be readily identified empirically. Additionally, suitable size and sequences of linker sequences also can be determined by conventional computer modeling techniques based on the predicted size and shape of the TCR molecule.

A number of strategies can be employed to express TCR fusion complexes of the invention. For example, the TCR gene fusion construct described above can be incorporated into a suitable vector by known means such as by use of restriction enzymes to make cuts in the vector for insertion of the construct followed by ligation. The vector containing the gene construct is then introduced into a suitable host for expression of the TCR fusion peptide. See, generally, Sambrook et al., *supra.* Selection of suitable vectors can be made empirically based on factors relating to the cloning protocol. For example, the vector should be compatible with, and have the proper replicon for the host that is being employed. Further the vector must be able to accommodate the DNA sequence coding for the TCR fusion complex that is to be expressed. Suitable host cells include eukaryotic and prokaryotic cells, preferably those cells that can be easily transformed and exhibit rapid growth in culture medium. Specifically preferred hosts cells include prokaryotes such as *E*. *coli, Bacillus subtillus,* etc. and eukaryotes such as animal cells and yeast strains, e.g., S. *cerevisiae.* Mammalian cells are generally preferred, particularly J558, NSO, SP2-O or CHO. Other suitable hosts include, e.g., insect cells such as Sf9. Conventional culturing conditions are employed. See Sambrook, *supra.* Stable transformed or transfected cell lines can then be selected. Cells expressing a TCR fusion complex of the invention can be determined by known procedures. For example, expression of a TCR fusion complex linked to an immunoglobulin can be determined by an ELISA specific for the linked immunoglobulin and/or by immunoblotting.

As mentioned generally above, a host cell can be used for preparative purposes to propagate nucleic acid encoding a desired fusion protein. Thus a host cell can include a prokaryotic or eukaryotic cell in which production of the fusion protein is specifically intended. Thus host cells specifically include yeast, fly, worm, plant, frog, mammalian cells and organs that are capable of propagating nucleic acid encoding the fusion. Non-limiting examples of mammalian cell lines which can be used include CHO dhfr-cells (Urlaub and Chasm, Proc. Natl. Acad. Sci. USA, 77:4216 (1980)), 293 *cells* (Graham et al., J Gen. Virol., 36:59 (1977)) or myeloma cells like SP2 or NSO (Galfre and Milstein, Meth. Enzymol., 73(B):3 (1981)).

Host cells capable of propagating nucleic acid encoding a desired fusion protein encompass non-mammalian eukaryotic cells as well, including insect (e.g., *Sp. frugiperda*), yeast (e.g., *S. cerevisiae, S. porribe, P. pastoris., K. lactis, H. polymorpha*; as generally reviewed by Fleer, R., Current Opinion in Biotechnology, 3(5):486496 (1992)), fungal and plant cells. Also contemplated are certain prokaryotes such as E. coli and Bacillus.

Nucleic acid encoding a desired fusion protein can be introduced into a host cell by standard techniques for transfecting cells. The term "transfecting" or "transfection" is intended to encompass all conventional techniques for introducing nucleic acid into host cells, including calcium phosphate co-precipitation, DEAE-dextran-mediated transfection, lipofection, electroporation, microinjection, viral transduction and/or integration. Suitable methods for transfecting host cells can be found in Sambrook et al. *supra,* and other laboratory textbooks.

The present invention further provides a production process for isolating a fusion protein of interest. In the process, a host cell (e.g., a yeast, fungus, insect, bacterial or animal cell), into which has been introduced a nucleic acid encoding the protein of the interest operatively linked to a regulatory sequence, is grown at production scale in a culture medium in the presence of the fusion protein to stimulate transcription of the nucleotides sequence encoding the fusion protein of interest. Subsequently, the fusion protein of interest is isolated from harvested host cells or from the culture medium. Standard protein purification techniques can be used to isolate the protein of interest from the medium or from the harvested cells. In particular, the purification techniques can be used to express and purify a desired fusion protein on a large-scale (i.e. in at least milligram quantities) from a variety of implementations including roller bottles, spinner flasks, tissue culture plates, bioreactor, or a fermentor.

An expressed TCR fusion complex can be isolated and purified by known methods. Typically the culture medium is centrifuged and then the supernatant is purified by affinity or immunoaffinity chromatography, e.g. Protein-A or Protein-G affinity chromatography or an immunoaffinity protocol comprising use of monoclonal antibodies that bind the expressed fusion complex such as a linked TCR or immunoglobulin region thereof. The fusion proteins of the present invention can be separated and purified by appropriate combination of known techniques. These methods include, for example, methods utilizing solubility such as salt precipitation and solvent precipitation, methods utilizing the difference in molecular weight such as dialysis, ultra-filtration, gel-filtration, and SDS-polyacrylamide gel electrophoresis, methods utilizing a difference in electrical charge such as ion-exchange column chromatography, methods utilizing specific affinity such as affinity chromatograph, methods utilizing a difference in hydrophobicity such as reverse-phase high performance liquid chromatograph and methods utilizing a difference in isoelectric point, such as isoelectric focusing electrophoresis, metal affinity columns such as Ni-NTA. See generally Sambrook et al. and Ausubel et al. *supra* for disclosure relating to these methods.

It is preferred that the fusion proteins of the present invention be substantially pure. That is, the fusion proteins have been isolated from cell substituents that naturally accompany it so that the fusion proteins are present preferably in at least 80% or 90% to 95% homogeneity (w/w). Fusion proteins having at least 98 to 99% homogeneity (w/w) are most preferred for many pharmaceutical, clinical and research applications. Once substantially purified the fusion protein should be substantially free of contaminants for therapeutic applications. Once purified partially or to substantial purity, the soluble fusion proteins can be used therapeutically, or in performing *in vitro* or *in vivo* assays as disclosed herein. Substantial purity can be determined by a variety of standard techniques such as chromatography and gel electrophoresis.

Truncated soluble single-chain TCR fusion complexes of the invention contain a TCR molecule that is sufficiently truncated so the TCR fusion complex can be secreted into culture medium after expression. Thus, a truncated TCR fusion complex will not include regions rich in hydrophobic residues, typically the transmembrane and cytoplasmic domains of the TCR molecule. Thus, for example, for a preferred truncated DR1 TCR molecule of the invention, preferably from about residues 199 to 237 of the β chain and from about residues 193 to 230 of the α chain of the TCR molecule are not included in the truncated TCR fusion complex.

The term "misfolded" as it relates to the fusion proteins is meant a protein that is partially or completely unfolded (i.e. denatured). A fusion protein can be partially or completely misfolded by contact with one or more chaotropic agents as discussed below. More generally, misfolded fusion proteins disclosed herein are representative of a high Gibbs free energy (ΔG) form of the corresponding native protein. Preferred are native fusion protein which is usually correctly folded, it is fully soluble in aqueous solution, and it has a relatively low ΔG. Accordingly, that native fusion protein is stable in most instances.

It is possible to detect fusion protein misfolding by one or a combination of conventional strategies. For example, the misfolding can be detected by a variety of conventional biophysical techniques including optical rotation measurements using native (control) and misfolded molecules.

By the term "soluble" or similar term is meant that the fusion molecule and particularly a fusion protein that is not readily sedimented under low G-force centrifugation (e.g. less than about 30,000 revolutions per minute in a standard centrifuge) from an aqueous buffer, e.g., cell media. Further, the fusion molecule is soluble if the it remains in aqueous solution at a temperature greater than about 5-37°C and at or near neutral pH in the presence of low or no concentration of an anionic or non-ionic detergent. Under these conditions, a soluble protein will often have a low sedimentation value e.g., less than about 10 to 50 svedberg units.

Aqueous solutions referenced herein typically have a buffering compound to establish pH, typically within a pH range of about 5-9, and an ionic strength range between about 2mM and 500mM. Sometimes a protease inhibitor or mild non-ionic detergent is added. Additionally, a carrier protein may be added if desired such as bovine serum albumin (BSA) to a few mg/ml. Exemplary aqueous buffers include standard phosphate buffered saline, tris-buffered saline, or other well known buffers and cell media formulations.

The present TCR fusion and conjugate complexes are suitable for in *vitro* or *in vivo* use with a variety of cells that are infected or that may become infected by one or more diseases.

As an illustration of the use of the TCR fusion/conjugate therapeutics, a cultured cell can be infected by a pathogen of a single serotype. The infected cell is then contacted by a specified fusion protein *in vitro.* As discussed previously, the fusion protein is configured so that the toxic domain is presented to the infected cell by the association of the TCR. After providing for introduction of the bioactive molecule to the cell (generally less than about 30 minutes), the cells are allowed to cause a desired effect for a time period of about up to about 2 to 24 hours, typically about 18 hours. After this time, the cells are washed in a suitable buffer or cell medium and then evaluated for viability. The time allotted for cell killing or injury by the fusion protein will vary with the particular effector molecule chosen. However viability can often be assessed after about 2 to 6 hours up to about 24 hours. As will be explained in more detail below, cell viability can be readily measured and quantified by monitoring uptake of certain well-known dyes (e.g., trypan blue) or fluors.

Cells transduced by the fusion molecules of the present invention can be assayed for viability by standard methods. In one approach, cell viability can be readily assayed by measuring DNA replication following or during transduction. For example, a preferred assay involves cell uptake of one or more detectably-labeled nucleosides such as radiolabelled thymidine. The uptake can be conveniently measured by several conventional approaches including trichloroacetic acid (TCA) precipitation followed by scintillation counting. Other cell viability methods include well know trypan blue exclusion techniques.

The TCR molecules of the fusion complexes of the invention suitably correspond in amino acid sequence to naturally occurring TCR molecules, e.g. TCR molecules of a human, mouse or other rodent, or other mammal.

Accordingly, a therapeutic composition for treatment of an autoimmune or inflammatory response would include a TCR complex which comprises a T cell receptor antagonist effector molecule that is an interleukin-2 (IL-2). A "truncated" soluble TCR complex is administered, i.e. the TCR complex does not contain a transmembrane portion. The effector molecule of the administered soluble TCR fusion complex can be selected that are specific for certain cells or specific to generate a desired result. Such effector molecule can be readily identified and selected by the methods of one of skill in the art. A TCR fusion complex that contains an effector peptide that is a T cell receptor antagonist or partial agonist is particularly useful for treatment of allergies and autoimmune diseases such as multiple sclerosis, insulin-dependent diabetes mellitus and rheumatoid arthritis.

Another composition of the invention for use in induction of an immune response provides for the administration of an effective amount of one or more TCR fusion complexes of the invention in the presence of any costimulatory effector molecule such as a cytokine to thereby induce a desired immune response at the location of the presented antigen which binds the TCR. The TCR fusion complex is a truncated form and is administered as a soluble protein as described above. Treatment with these complexes will comprise administration to a mammal an effective amount of a DNA sequence that comprises a DNA vector encoding the TCR fusion complex of the invention and an effector molecule that is an interleukin-2 (IL-2).

Different therapies of the invention also may be used in combination as well as with other known therapeutic agents such as anti-inflammatory drugs to provide a more effective treatment of a disorder. For example, immunosuppressive TCR fusion complexes that can be used in combination with anti-inflammatory agents such as corticosteroids and nonsteroidal drugs for the treatment of autoimmune disorders and allergies.

Compounds of the invention will be especially useful to a human patient who has or is suspected of having a malignant disease, disorder or condition. Compounds of the invention will be particularly useful in targeting particular tumor antigens in human patients. Specific examples of diseases which may be treated in accordance with the invention include cancers, e. g. breast, prostate, etc; viral infections, e.g. HCV, HIV, etc. as well as other specific disorders of conditions mentioned herein.

Without wishing to be bound by theory, it is believed the multiple and distinct covalently linked compounds of this invention (i.e. at least one identified anti-cancer drug in combination with at least one TCR) can significantly enhance efficacy of the anti-cancer drug, e.g., by increasing targeting of drug to target antigen in subject individuals.

Moreover, by virtue of the covalent linkage, the conjugates of the invention present the anti-cancer drug and the TCR to the subject cell essentially simultaneously, an effect that may not be readily achieved by administering the same compounds in a drug "cocktail" formulation without covalently linking the compounds.

It also has been reported that treatment with one drug can in turn sensitize a patient to another drug. Accordingly, the essentially simultaneous presentation to the subject cell of an anti-cancer drug and TCR via a conjugate of the invention may enhance drug activity, e.g., by providing synergistic results and/or by enhancing production an immune response.

Administration of compounds of the invention may be made by a variety of suitable routes including oral, topical (including transdermal, buccal or sublingal), nasal and parenteral (including intraperitoneal, subcutaneous, intravenous, intradermal or intramuscular injection) with oral or parenteral being generally preferred. It also will be appreciated that the preferred method of administration and dosage amount may vary with, for example, the condition and age of the recipient.

Compounds of the invention may be used in therapy alone or in conjunction with other medicaments such those with recognized pharmacological activity to treat the desired indications. Exemplary medicaments include recognized therapeutics such as surgery, radiation, chemotherapy and other forms of immunotherapy (e.g. vaccines, antibody based therapies). The compounds of this invention can be administered before, during or after such therapies as needed.

While one or more compounds of the invention may be administered alone, they also may be present as part of a pharmaceutical composition in mixture with conventional excipient, i.e., pharmaceutically acceptable organic or inorganic carrier substances suitable for parenteral, oral or other desired administration and which do not deleteriously react with the active compounds and are not deleterious to the recipient thereof. Pharmaceutical compositions of the invention in general comprise one or more TCR fusion complexes of the invention or DNA constructs coding for such fusion complexes together with one or more acceptable carriers. The carriers must be "acceptable" in the sense of being compatible with other ingredients of the formulation and not deleterious to the recipient thereof. Suitable pharmaceutically acceptable carriers include but are not limited to water, salt solutions, alcohol, vegetable oils, polyethylene glycols, gelatin, lactose, amylose, magnesium stearate, talc, silicic acid, viscous paraffin, perfume oil, fatty acid monoglycerides and diglycerides, petroethral fatty acid esters, hydroxymethyl-cellulose, polyvinylpyrrolidone, etc. The pharmaceutical preparations can be sterilized and if desired mixed with auxiliary agents, e.g., lubricants, preservatives, stabilizers, wetting agents, emulsifiers, salts for influencing osmotic pressure, buffers, colorings, flavorings and/or aromatic substances and the like which do not deleteriously react with the active compounds.

For parenteral application, particularly suitable are solutions, preferably oily or aqueous solutions as well as suspensions, emulsions, or implants, including suppositories. Ampules are convenient unit dosages.

For enteral application, particularly suitable are tablets, dragees or capsules having talc and/or carbohydrate carrier binder or the like, the carrier preferably being lactose and/or corn starch and/or potato starch. A syrup, elixir or the like can be used wherein a sweetened vehicle is employed. Sustained release compositions can be formulated including those wherein the active component is protected with differentially degradable coatings, e.g., by microencapsulation, multiple coatings, etc.

Therapeutic compounds of the invention also may be incorporated into liposomes. The incorporation can be carried out according to known liposome preparation procedures, e.g. sonication and extrusion. Suitable conventional methods of liposome preparation are also disclosed in e.g. A.D. Bangham et al., J. Mol. Biol., 23:238-252 (1965); F. Olson et al., Biochim. Biophys. Acta, 557:9-23 (1979); F. Szoka et al., Proc. Nat. Acad. Sci., 75:4194-4198 (1978); S. Kim. et al., Biochim. Biophys. Acta, 728:339-348 (1983); and Mayer et al., Biochim. Biophys. Acta, 858:161-168 (1986).

The disclosure also provides methods for invoking an immune response in a mammal such as a human, including vaccinating a mammal such as a human against an infectious agent or a targeted disorder such as cancer.

These methods comprise administering to a mammal an effective amount of a DNA sequence that comprises a DNA vector that codes for a TCR fusion complex of the invention. Preparation of expression vectors of TCR fusion complexes is described above and in the Examples which follow. Methods for administration of plasmid DNA, uptake of that DNA by cells of the administered subject and expression of protein has been reported. See Ulmer, J.B., et al., Science (1993) 259: 1745-1749.

DNA vectors that encode TCR fusion complexes of the invention are suitably administered to a mammal including a human preferably by intramuscle injection. Administration of cDNA to skeletal muscle of a mammal with subsequent uptake of administered expression vector by the muscle cells and expression of protein encoded by the DNA has been described by Ulmer et al. and represents an exemplary protocol [Ulmer, J.B., et al., Science 259: 1745-1749]. The optimal dose for a given therapeutic application can be determined by conventional means.

In addition to treatment of human disorders, TCR fusion and conjugate complexes of the invention and DNA constructs of the invention that encode such fusion complexes will have significant use for veterinary applications, e.g., treatment of disorders of livestock such as cattle, sheep, etc. and pets such as dog and cats.

It will be appreciated that actual preferred amounts of a given TCR fusion complex of the invention or DNA construct coding for same used in a given therapy will vary according to the particular active compound or compounds being utilized, the particular compositions formulated, the mode of application, the particular site of administration, the patient's weight, general health, sex, etc., the particular indication being treated, etc. and other such factors that are recognized by those skilled in the art including the attendant physician or veterinarian. Optimal administration rates for a given protocol of administration can be readily determined by those skilled in the art using conventional dosage determination tests conducted e.g. with regard to the foregoing guidelines and the assays disclosed herein.

The following non-limiting examples are illustrative of the invention.

### Example 1

### Construction of 264 single-chain (sc) TCR

The T cell clone, 264, recognizes a peptide fragment (aa 264-272; LLGRNSFEV) of the human wild-type tumor suppresser protein p53 restricted by HLA-A2.1. The T cell receptor gene was cloned into a three domain single-chain format previously shown to produce soluble TCR and functional receptor molecules.

In brief, mRNA was isolated from the T cell clone and cDNA was made using the Marathon cDNA Amplification Kit (Clontech). Sequencing of cDNA clones identified two distinct V alpha chains (Valpha 3 and V alpha 13) and a single V beta chain (V beta 3). The cDNA was used as a template in polymerase chain reaction (PCR) with primers KC228 and KC229 or KC226 and KC227 to produce 5'SfiI-3'SpeI V alpha 3 or V alpha 13 fragments respectively. The same DNA was then used as a PCR template with primers PRIB4 and KC176 to generate a 5XhoI-3XmaI V beta C beta chain fragment. The C beta chain was truncated just before the cysteine residue at amino acid 127 of the full length C beta chain.

The alpha and beta chain fragments were cloned into the pGEM-T Easy Vector System (Promega) for DNA sequence determination. Correct fragments were restriction digested and cloned into expression vector pKC60 (described previously in pending U.S. patent application no. 08/813,731) to create two V alpha-(G₄S)₄ V beta C beta scTCR molecules, 264-A (with V alpha 3) and 264-B (with V alpha 13).

The DNA constructs described above (264-A and 264-B) were reamplified by PCR with primers ET-TCRF1 and KC170 or ET-TCRF2 and KC170, respectively, to generate 5'AgeI-3'ClaI DNA fragments. The fragments were cloned into the pGEM-T Easy Vector System for DNA sequence determination.

The 5'AgeI-3'ClaI fragments were then used as the template DNA in PCR with primers KC232 and KC208 or KC231 and KC208, respectively, to produce 5AgeI-3HpaI DNA fragments for cloning into the CD3 zeta fusion molecule (described below) and eventually the 264 IL-2 fusion molecule (described below).

### Example 2

### Construction of the CD3 zeta Fusion Vector

To determine which of the two V alpha chains was functional, both the 264-A and 264-B scTCR were expressed as CD3 zeta fusion molecules.
Construction of a shuttle vector has been previously described in pending U.S. application no. 09/422,375.

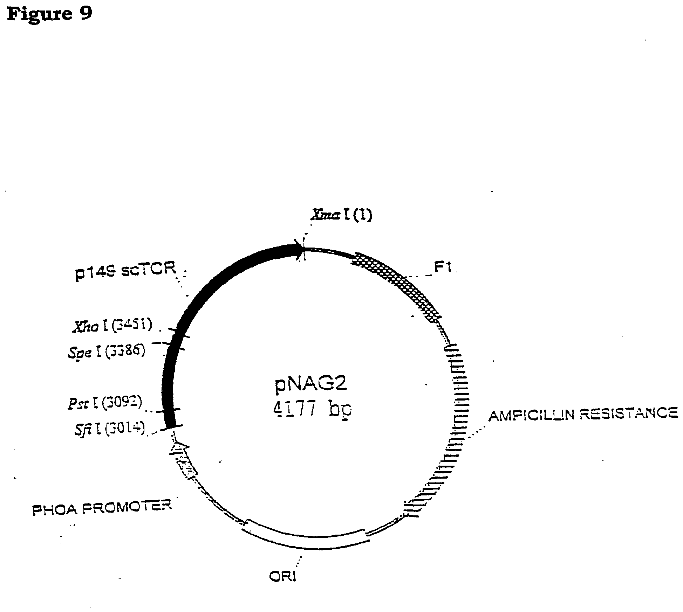

Briefly, alpha and beta chain TCR fragments were cloned into the into the expression vector pKC60 to create a V alpha-(G₄ S)₄ V beta C beta scTCR molecule. The new vector was named pNAG2 (Fig. 9). pNAG2 was then reamplified by PCR with primers KC203 and KC208 to generate a 5'AgeI-3'HpaI/BspEI/NruI/ClaI DNA fragment. The scTCR fragment was cloned into the pGEM-T Easy Vector System and this new pGEM-based vector was then used as a "shuttle vector" for introduction of other DNA fragments to create a bispecific sc molecule.

Sc-Fv DNA was then restriction digested and cloned into the "shuttle vector" downstream of the scTCR. To connect the scTCR and scSc-Fv together as a single-chain fusion protein, the "shuttle vector" was digested with the appropriate restriction enzymes to drop out the previous linker DNA fragment and allow for ligation of linker sequences between the scTCR and the Sc-Fv.

In the "shuttle vector" design outlined above, a stop codon and splice site were introduced between the NruI and ClaI restriction sites as part of the PCR amplification of the scTCR with "back" primer KC208. To aid in downstream purification of the bispecific sc protein, a set of annealed oligos (KC237 and KC238) was designed to introduce a 3' EE tag (EEEEYMPME) SEQ ID NO: 2 with stop codon and splice site. The annealed oligo pair was cloned 5'NruI-3'ClaI into the "shuttle vector" already encoding for the complete bispecific sc molecule.

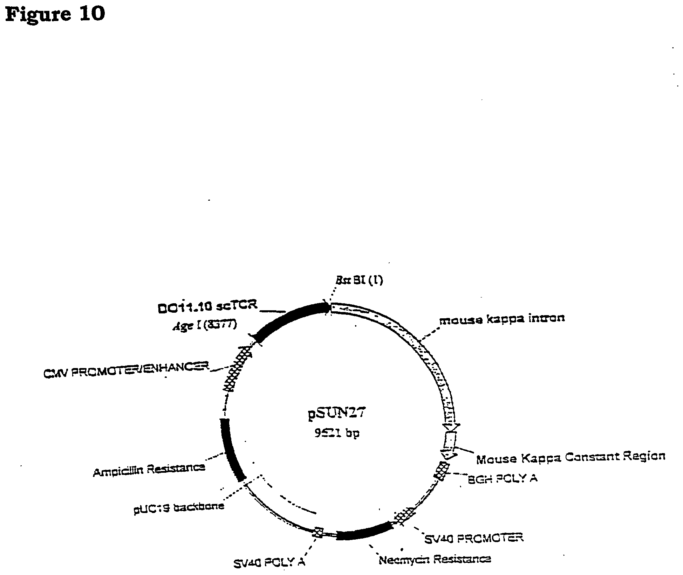

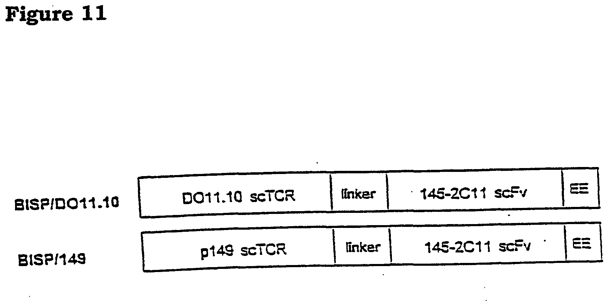

After cloning the scTCR, Sc-Fv, linker, and tag DNA fragments into the "shuttle vector" to complete the bispecific sc molecule design, the DNA was restriction digested (AgeI-ClaI) and cloned into the mammalian cell expression vector pSUN27 (Fig. 10) (previously described in the pending U.S. Application Serial No. 08/943,086 to create pBISP/149 (Fig. 11).

### Construction of the CD3 zeta Fusion Vector

In brief, murine cDNA was used as the template in polymerase chain reaction (PCR) with primers KC312 and KC304 to produce a 5'HpaI-3'ClaI murine CD3 zeta fragment.

The murine CD3 zeta fragment was cloned into the pGEM-T Easy Vector System for DNA sequence determination. The correct fragment was restriction digested and cloned into the "shuttle vector", effectively removing the existing linker, scFV, and EE tag.

After cloning the CD3 zeta gene into the "shuttle vector ", the DNA was digested AgeI-HpaI to allow for ligation with the 264-A and 264-B scTCR fragments (described above), creating two new scTCR/CD3 zeta fusions. Lastly, the new DNA preparations were restriction digested (AgeI-ClaI) and cloned into the mammalian cell expression vector pSUN28 (pBISP/DO 11. 10 vector), Fig. 11 previously described in pending patent U.S. application no. 09/422,375.

### Example 3

### Expression of 264 scTCR/CD3 Zeta Fusion Molecules

Jurkat cells were prepared for transfection by washing with cold DPBS. The cells were resuspended in DPBS and mixed with 20 ug of PvuI linearized 264-A/CD3 zeta or 264-B/CD3 zeta DNA.. After five minutes on ice, the cells were electroporated using a Gene Pulser (BioRad) set to deliver one pulse of 250 volts, 960 u Fd or 0.25 u Fd. The pulsed cells were placed on ice for five minutes. The cells were diluted into 10 ml of 10% IMDM medium (IMDM, 10% FBS, 2mM glutamine) and grown in a T-25cm² TC flask overnight at 37C with 5% CO₂. The next day, the cells were plated in 96 well plates with selective medium (10% IMDM plus 1.0 mg/ml G418). After 1 week, the concentration of G418 was increased to 2 mg/ml. The growing colonies were refed approximately two weeks after transfection and screened about one week later.

The transfected Jurkat cells were screened for surface expression of scTCR using flow cytometry analysis. Positive transfectants were identified by staining with a fluorescent-tagged mAb (H57-597) which detects a portion of the C beta domain of murine TCR.

### Example 4

### Identification of the Correct 264 scTCR V alpha Domain

Transfected Jurkat cells which expressed either the 264-A or 264-B version of the CD3 zeta fusion molecule were used in a cell activation assay. In the assay, the HLA-A2 presenting cell line T2 was used as the APC. The T2 cells were loaded with 264 peptide (or irrelevant peptide) overnight at 37C with 5% CO2. The following day, the transfected Jurkat lines were added and allowed to interact with the peptide-pulsed APCs overnight.

Specific stimulation of the transfectants by 264-loaded APCs was assessed using an IL-2 ELISA. An anti-human IL-2 mAb was coated passively overnight on a 96 well plate. The plate was washed and blocked with 10% FBS/DPBS for 1 hour. The blocking reagent was flicked out and supernatants from the assay were added to the plate for 1 hour at 37C. After washing, the bound IL-2 was detected using another anti-IL-2 mAb conjugated to biotin. Following 45 minutes at 37C, the plate was washed and strepavidin-HRP was added for 15 minutes. Finally, the plate was washed and developed using ABTS substrate. Absorbance was read at 405 nm.

Based on the cell activation assay, the V alpha 3 domain is functional. Only the 264-A molecule was stimulated to produce IL-2 in the presence of 264 peptide-loaded APCs.

### Example 5

### Construction of the 264 scTCR/IL-2 Fusion Molecule

To generate the scTCR/IL-2 fusion molecule, the human IL-2 gene needed to be cloned into a DNA expression vector.

In brief, total RNA was isolated from human Jurkat cells using the Mini Total RNA Kit (Qiagen) and Qiashredder (Qiagen). The RNA was concentrated and used in a reaction with reverse transcriptase and a specific back primer, KC328B, to generate cDNA. The cDNA was used as the template in PCR with primers KC327B and KC328B to produce a 5'BspI-3'NruI human IL-2 gene fragment.

The human IL-2 fragment was cloned into the pGEM-T Easy Vector System for sequence determination. The correct fragment was restriction digested and cloned into the "shuttle vector", effectively removing the existing scFv gene.

The "IL-2 modified shuttle vector" was then restriction digested (BspI-NruI) and the scTCR (described above) was ligated in to complete the scTCR/IL-2 design. Finally, the DNA was cut AgeI-ClaI and cloned into the mammalian cell expression vector pSUN28.

### Example 6

### Construction of the 149 scTCR/IL-2 Fusion Molecule

To create the 149 scTCR (described in detail in patent application 09/422,375) version of the IL-2 fusion, the 149 scTCR was cut out of the "shuttle vector" (see example 7 of patent application 09/422,375) as an 5'AgeI-3'HpaI fragment and then ligated into the "IL-2 modified shuttle vector" (described above). The 149 scTCR/IL-2 fragment was then restriction digested (AgeI-ClaI) and cloned into the mammalian cell expression vector pSUN28.

### Example 7

### Expression of the scTCR/IL-2 Fusion Molecules

CHO cells were prepared for transfection by washing with cold DPBS. The cells were resuspended in DPBS and mixed with 20 ug of PvuI linearized 264 scTCR/IL-2 or 149 scTCR/IL-2. After five minutes on ice, the cells were electroporated using a Gene Pulser set to deliver one pulse of 250 volts, 960 u Fd or 0.25 u Fd. The pulsed cells were placed on ice for five minutes. The cells were diluted into 10 ml of 10% IMDM medium (IMDM, 10% FBS, 2mM glutamine) and grown in a T-25cm²TC flask overnight at 37C with 5% CO₂. The next day, the cells were plated in 96 well plates with selective medium (10% IMDM plus 1 mg/ml G418) and refed after approximately 7 days.

Transfectants were screened for expression of soluble fusion molecules in an ELISA assay format. An anti-human IL-2 antibody was passively coated overnight onto a 96 well plate. On assay day, the plates were blocked with 10% FBS/PBS for one hour. The wells were washed and supernatant from the transfectants was added to the plate. After incubating and washing, biotinylated anti-C beta mAb H57-597 (cell line was purchased from ATCC) was added to the plate, followed by washing and incubation with streptavidin-HRP. Positive wells were identified by the addition of TMB substrate, quenched with IN sulfuric acid, and read at an absorbance of 450nM. A small number of positive clones were selected for expansion and limiting dilution cloning was carried out to establish stable transfected cell lines.

Transfectants could also be screened for the expression of fusion molecules in an ELISA assay format using mAbs which specifically recognize each of the scTCRs followed by detection with biotinylated anti-C beta mAb and streptavidin-HRP. For the 149 fusion molecule, a conformational mAb to the V alpha domain (B20.1, Pharmagen) was used as the coating antibody. The 264 fusion molecule could be detected using the a conformational mAb to its V beta domain (KJ25, Pharmingen).

### Example 8

### Purification of scTCR/IL-2 fusion protein.

TCR/IL-2 fusion proteins were purified from transfectant supernatant using standard affinity chromatography methods. The fusion proteins were applied to an anti-TCR C□□ specific CNBr-coupled agarose column for enrichment. In brief, supernatant was passed over the column bed one time. After washing with PBS, the bound protein was eluted off the column by the addition of low pH glycine buffer (pH3.0) and immediately neutralized by the addition of a 1 to 10 dilution of 2M Tris, pH 8.0. The purified protein was then buffered exchanged into PBS using a 30kD MW cut-off concentration unit. The final protein concentration was determined by an OD280 reading. Coomassie blue staining of the purified protein (Fig. 2) and immunoblot analysis of the purified protein (Fig. 2) shows enrichment for the 264 scTCR-IL-2 fusion protein.

### Example 9

### CTLL-2 proliferation assay.

The IL-2 dependent murine T cell line, CTLL-2, was used to evaluate the IL-2 activity of the 264 scTCR-IL-2 fusion protein using a non-radioactive cell proliferation assay. The 264 scTCR-D fusion protein was used in the assay as a negative control. Briefly, CTLL-2 cells used for the assay were seeded at 104 cells/ml and allowed to grow for 48 hrs in order to deplete residual IL-2. Over the 48 hr period the cells grew to a density of 1.15 x 105cells/ml. Cells were then harvested and washed several times using 10% IMDM (w/o IL-2) to remove any remaining IL-2. A 96 well flat bottom plate was used for the assay. First, 50 ul of media (10% IMDM), media w/IL-2 or purified 264-IL-2 or 264-k fusion protein was added to each well. The CTLL-2 cells were added to wells at 105cells/50ul. An IL-2 standard was run on the same plate. The plate was incubated overnight at 37oC with 5%CO2. The following day, cell death was clearly evident using a microscope. Cell proliferation/viability was assessed using the Celltiter Assay. The CellTiter96 assay is an aqueous non-radioactive cell proliferation assay marketed by Promega Corp. The assay is composed of solutions of a novel tetrazolium compound, MTS, and an electron coupling reagent; PMS. MTS is bioreduced by cells into a formazan that is soluble in tissue culture medium. The absorbance of the formazan at 490nm can be measured directly from the 96 well assay plates without additional processing. The conversion of MTS into the aqueous soluble formazan is accomplished by dehydrogenase enzymes found in metabolically active cells. The quantity of formazan product measured by the amount of 490nm absorbance is directly proportional to the number of living cells in culture. The 264/IL-2 and 264-k fusion proteins were tested for activity by diluting from 1.25 µg/well to 0.0098 µg/well.

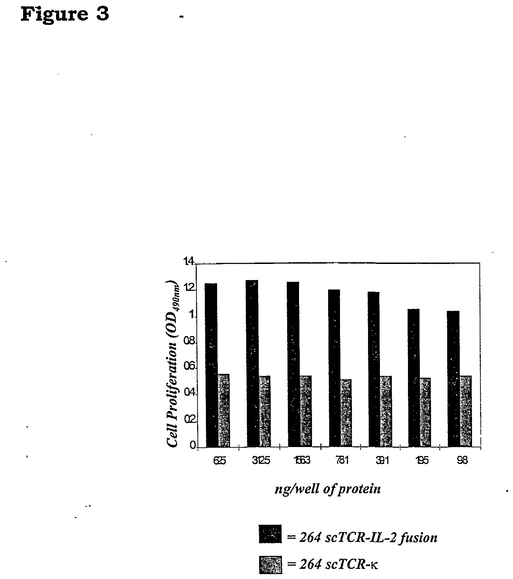

The results from one experiment are shown in Figure 3. The IL-2 dependent murine T cell line, CTLL-2, was used to evaluate the IL-2 activity of the 264 scTCR-IL-2 fusion protein using a non-radioactive cell proliferation assay. The 264 scTCR-κ fusion protein was used in the assay as a negative control.

### Example 10

Staining of peptide-pulsed cells with the 264 scTCR-IL-2 fusion protein demonstrates a functional scTCR.

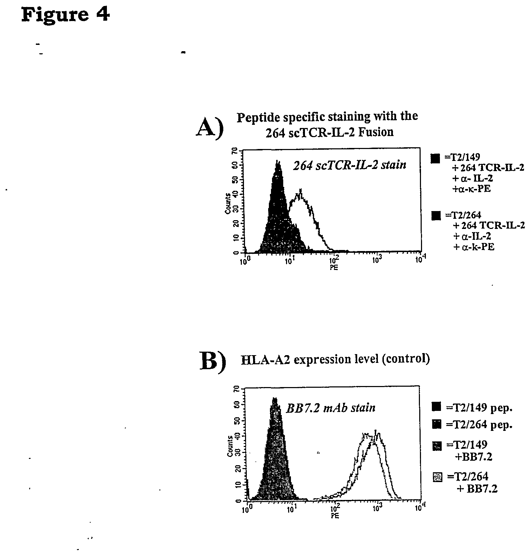

Flow cytometry and immunofluoresence staining were used to show direct binding of the fusion protein via its TCR to peptide/HLA-A2 complexes on the surface of the human B lymphoid cell line T2 (Fig. 4)
A) Staining of 149 or 264 peptide pulsed T2 cells with 0.5µg (10µg/ml) of 264 scTCR-IL-2 fusion protein. The 264 scTCR-IL-2 fusion protein binds specifically to T2 cells displaying the 264 peptide but not the 149 peptide.
B) T2 cells were pulsed with 50µg of either 149 or 264 peptide. To evaluate A2 loading of each peptide, the cells were stained with the HLA-A2 specific mAb BB7.2 (0.05 µg) after overnight incubation of the cells with peptide. The results from these experiments show an equivalent level of HLA-A2 surface expression on T2 cells pulsed with either peptide indicating efficient HLA-A2 binding of both peptides.

### Example 11

Cell-Cell conjugation mediated specifically by the 264 scTCR-IL-2 fusion protein.

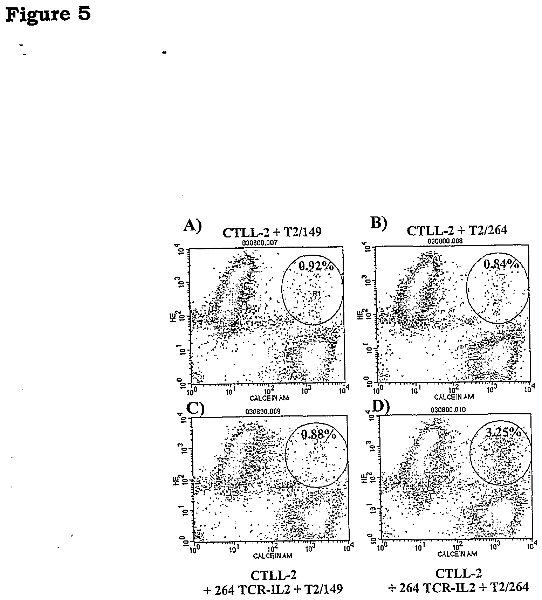

In this experiment, T2 cells pulsed with either the 149 or 264 peptide. CTLL-2 cells were hydrodiethidium (HE) labeled and incubated for 20 minutes at RT with an equal number of calcein-AM labeled T2 cells pulsed with either 50 µg of 149 or 264 peptide. Figure 5 shows conjugation between cells when 1 µg of fusion protein was added to the incubation mixture containing CTLL-2 cells and 264 peptide-loaded T2 cells **(A; 3.25%).** In contrast, conjugate formation was not observed with the mixture that included the 149 peptide pulsed T2 cells **(B; 0.88%).** Cell samples were washed one time before analysis on the flow cytometer.

### Example 12

Construction of scTCR/IgG (murine) Fusion Molecules, have been previously described in pending U.S. application No. 09/422,375. (referential)

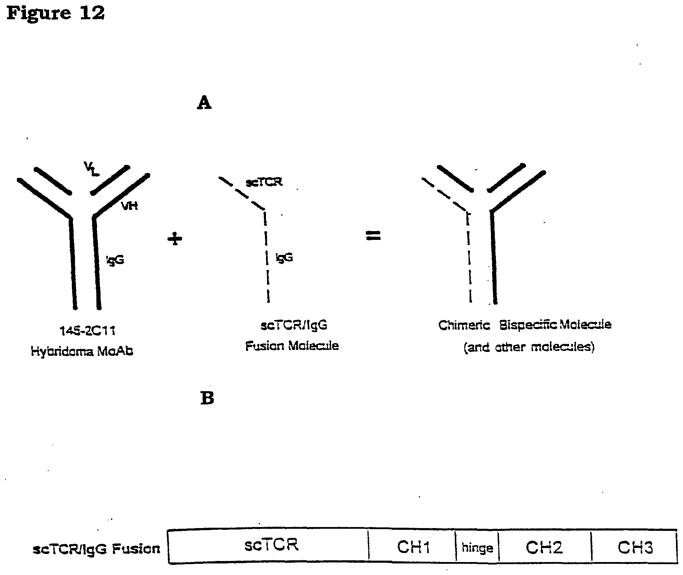

There has been recognition that the expression of the 145-2CII scSc-Fv alone, i.e. not as part of a bispecific sc molecule, is very low. Without wishing to be bound to theory, the low level of sc-Fv expression may be a limiting factor in the expression of bispecific molecules. Native 145-2C 11 hybridoma cell line was used as antibody source and cells were transfected with scTCR fused with murine IgG2b heavy chain (Fig. 12). The transfected hybridoma cell line should secrete some 145-2C11/scTCR chimeric molecules if the host's hamster IgG can pair efficiently with murine IgG2b heavy chain.

To clone the p149 scTCR as an IgG fusion, an internal EcoRI restriction site was first mutated using site-directed mutagenesis. Briefly, a pair of complimentary oligonucleotides, KC293 and KC294, were designed containing the desired mutation. The pNAG2 DNA construct was amplified by PCR with the primers using *Pfu* DNA polymerase. The resulting PCR product was digested with *DpnI* which digests the parental DNA template, leaving the mutated DNA intact. The mutated scTCR DNA was sequenced and then reamplified by PCR with primers KC276 and KC268 to generate a 5'NruI-3'EcoRI DNA fragment. The mutated scTCR DNA was cloned into the pGEM-T Easy Vector System for DNA sequence determination. The correct scTCR DNA was restriction digested and cloned into the mammalian cell expression vector pSUN7 to create the pl49 scTCR/IgG fusion molecule.

### Construction of DO 11.10 scTCR/IgG Fusion Molecule

The pKC60 DNA construct was reamplified by PCR with primers KC275 and KC268 to generate a 5'NruI-3'EcoRI DNA fragment. The scTCR fragment was cloned into the pGEM-T Easy Vector System for DNA sequence determination. The correct scTCR DNA was restriction digested and cloned into the mammalian cell expression vector pSUN7 to create the DO 11.10 scTCR/IgG fusion molecule (See Figures 12A/12B).

### Construction of the Murine IgG2b Expression Vector

The construction of the murine IgG2b (heavy chain) expression vector was as follows. The backbone of the vector was the plasmid pCDNA3 (Invitrogen). The plasmid was cut with HindIII and XhoI and a "light chain polylinker" DNA fragment was inserted to create the starting "light chain vector" pCDNA3.LCPL. This linker contained the restriction sites HindIII, KpnI, ClaI, PmlI, EcoRV, XmaI, BarnHI, and XhoI to facilitate subsequent cloning steps. A SmaI-BclI DNA fragment containing a light chain leader, mouse anti-CKMB kappa light chain genomic fragment, and 3' UTR was cloned into the EcoRV-BarnHI sites of pCDNA3.LCPL. Mutagenesis was then performed to eliminate an NruI MluI, and BstBI site and to introduce an NheI and BarnHI site to create the plasmid pCDNA3mut.LCPL.LCVK.

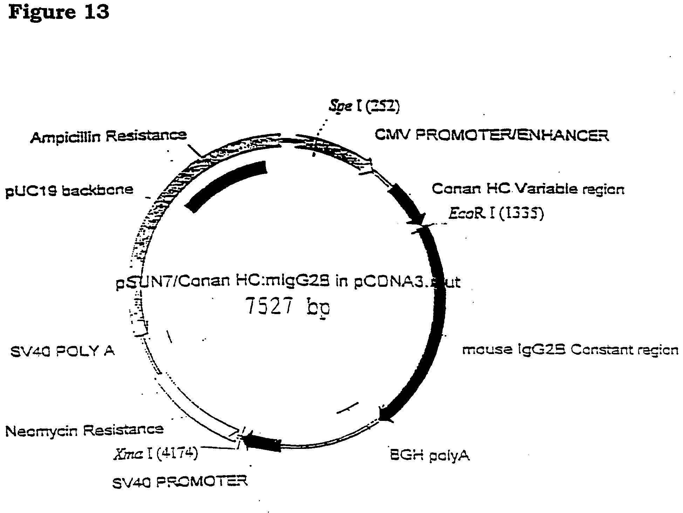

The "heavy chain vector" pCDNA3mut.HCPL was constructed from the pCDNA3mut.LCPL.LCVK plasmid by replacing the light chain expression region (HindIII-XhoI) with a "heavy chain polylinker" consisting of restriction sites HpaI, BspEI, EcoRV, KpnI, and XhoI. This plasmid was digested with EcoRv and KpnI. A SmaIKpnI digested DNA fragment containing a heavy chain leader and an anti-CKMB IgG2b mouse heavy chain genomic fragment (see Near et al., Molecular Immun., 1990) was then ligated into the EcoRV-KpnI digested plasmid. A KpnI-SaII oligonucleotide fragment containing a 3'UTR and a NotI site upstream of the SaII site was subsequently cloned into the KpnI-XhoI digested plasmid (knocking out the XhoI site) to create the plasmid pCDNA3mut.HCPL.HCV2b, also known as the murine IgG2b expression vector pSUN7 (Fig. 13).

### Example 13-Construction of scTCR/IgG (human) Fusion Molecules.(

### Cloning of scTCR 264 into pJRS355 and expression as an IgG1 fusion). (referential)

A DNA preparation of the 264 scTCR provided by Kim Card was used as a template for the PCR amplification of this scTCR construct. Reamplification of the scTCR was carried out using the primer set of 264 TCRls and KC268. The newly designed 264 TCRls sequence reads as follows, 5'-TTTCgTACgTCTTgTCCCAgTCAgTgACgCAgC-3' SEQ ID NO:3. This oligonucleotide has been designed with a *Bsi WI* restriction endonuclease site and a B6.2 leader. Takara ExTag polymerase was used in the amplification reaction following standard PCR protocol. The amplification profile was as follows, 96oC/2min for 1 cycle; 96oC/30sec, 62C/ 15sec, 72C/30sec for 5 cycles; and 96C/30sec, 68C/ lmin for 30 cycles. The proper MW (∼1.3Kb) DNA band was gel purified following the Clonetech protocol and cloned into Promega's pGem-T easy vector. After ligation and transformation into XL1-Blue cells, six clones were picked and screened by diagnostic PCR using two primers, KC 285 and KC 288, provided by Kim Card. Five clones out of six, produced a DNA band of the proper MW. DNA sequence analysis was carried out on two clones, scTCR264/pGem A and B, with each clone found to be correct. Double digest *(Bsi WI* and *Eco RI)* reactions were set up for clones A and B. The proper DNA fragments were gel purified and pooled together. The purified 264 scTCR was cloned into a previously prepared pJRS355 vector DNA. After ligation and transformation into XL1-Blue cells, two colonies were picked (A2 and B1). An *Alw NI* digest of their DNA showed the proper restriction pattern. Transient transfection using A2 DNA produced a 264 scTCr/IgG1 molecule as determined using an ELISA assay with antibodies specific to the TCR and to the IgG1 isotype.

### Example 14. Demonstration of anti-tumor effects of modified TCR in vitro.

In example 11 we showed the TCR/IL-2 fusion protein able to mediate conjugation between a T cell and an antigen presenting cell pulsed with the correct peptide. Now we are interested in whether the crosslinking mediated by the fusion protein results in the destruction of the target cell. To determine if indeed this is the case, we will use an *in vitro* killing assay. Briefly, effector cells are generated from isolated murine splenocytes and cultured for three days at 370C in 5% CO2 in the presence of stimulation with soluble recombinant human IL-2 (50 ng/ml) and anti-CD3D mAb (145-2C11; 10ng/ml). After three days in culture with stimulation, double staining of cells is carried out using anti-CD8 and anti-CD25 mAbs and flow cytometry. Detection of a double positive population is indicative of successful generation of effector CTL.

The killing assay is carried out using labeled target cells (e.g. peptide pulsed T2 cells, various carcinomas) with calcein-AM dye. Live cells (targets) incorporate the dye, are then washed and added to a 96 well plate containing effector cells and either the TCR/IL-2 fusion protein or positive or negative control proteins IL-2 and TCR-k fusion respectively. The ratios of effector to target cell will generally be 5:1, 10:1 and 20:1. The assay components are then incubated for 2 to 4 hours at 37oC and the release of calcein-AM to the culture supernatant is measured. The specific release of calcein-AM is measured or compared to the non-specific control of spontaneous released calcein-AM.

### Example 15

### In vivo Demonstration of anti-tumor effects of modified TCR.

In order to test the ability of the TCR/IL-2 fusion protein to facilitate elimination of human tumors that naturally express both A2 and p53, we will use a model in which such tumors are established in Nude and SCID mice. This model has also been used in Dr. Sherman's laboratory to test the efficacy of T cell clones and immunocytokines directed against human tumors. Tumors known to express p53 and to be specifically killed by 264 specific CTL will be tested for their ability to grow in Nude and SCID mice. Candidates include MDA-238, BT549, MCF-7, Caski (cervical carcinoma), and HepG2 cells. The cells (1x10⁶) will be implanted subcutaneously into Nude and SCID mice and allowed to establish for 7 days prior to treatment. Initially we will determine for each construct under evaluation, whether tumor growth is inhibited when mice receive the TCR-IL-2 fusion molecule alone. Although we anticipate that T cells will be required as effector cells for tumor elimination, other lymphoid cells present in the Nude and SCID due to leakiness may have effector function that can be triggered by the fusion protein, and this could inhibit tumor growth. This is particularly true in the presence of bispecific antibody molecules that have Fc□ or cytokines capable of stimulating nonlymphoid components of the innate immune system. Once we have determined the ability of the fusion protein to affect tumor growth, we will then test different T cell populations for effector function. The previous experiments in the murine tumor model will provide the information necessary to decide if naive or activated T cells will be delivered. For control purposes, we will deliver activated T cells as effectors into mice without concurrent TCR-IL-2 fusion treatment.

### Example 16

### Preparation of Doxorubicin (Dox) conjugates and in vitro characterization of anti-tumor properties. (referential)

The purpose of this example is to develop an immunoconjugate using the 264 scTCR-k and a cytotoxic drug such as Doxorubicin. Dox, a member of the anthracycline family of drugs, is one of the most potent anti-cancer drugs known but its clinical application has been limited due to its cardio-toxicity. An attempt to overcome the cardio-toxicity has been to attach the Dox to a carrier molecule, such as an anti-tumor mAb, to deliver the drug specifically to tumor sites. Results from studies in pre-clinical models have demonstrated that Dox-immunoconjugates can kill tumor cells more effectively with less toxicity than equivalent doses of the free drug.

In this example, we will prepare and purify a 264 scTCR-Dox conjugate and then carry out several studies with the immunoconjugate to characterize its tumor killing activity in *vitro* and *in vivo.* First, the optimal number of Dox molecules coupled to the scTCR-k fusion protein will be determined. The amount of Dox internalized by a cell will directly influence the rate and efficiency of tumor killing. Therefore, if we assume the number of peptide/MHC targets displayed on the tumor cell is limiting, then coupling increasing numbers of Dox molecules onto the scTCR may be desirable. However, the stability of the linkage between the scTCR and the Dox group will have to be sufficiently high enough to prevent non-specific cell cytotoxicity *in vivo* associated with shedding of Dox. Collectively, findings from these studies may be used to predict the performance of the TCR-Dox conjugate in pre-clinical studies.

## Claims

1. A soluble single-chain T cell receptor fusion complex comprising a single-chain T cell receptor polypeptide and an interleukin-2 (IL-2) polypeptide connected by a peptide linker.

2. The soluble single-chain T cell receptor fusion complex of claim 1 wherein the single-chain T cell receptor polypeptide is specific for recognition of a particular antigen.

3. The soluble single-chain T cell receptor fusion complex of claim 1 or 2 wherein the single-chain T cell receptor polypeptide is a heterodimer comprising a and b chain TCR.

4. The soluble single-chain T cell receptor fusion complex of claim 1-3 wherein the single-chain T cell receptor polypeptide a and b chains are linked through a non-covalent linkage.

5. A method of preparing a soluble single-chain T cell receptor fusion complex, the method comprising:
providing a single-chain T cell receptor polypeptide chain, or subfragment thereof;
providing an IL-2 polypeptide;
connecting the single-chain T cell receptor chain and the IL-2 polypeptide to a peptide linker; and
recovering the linked single-chain T cell receptor fusion polypeptide complex, thereby generating a soluble single-chain T cell receptor fusion complex.

6. The method of claim 5 wherein the single-chain T cell receptor polypeptide is specific for recognition of a particular antigen.

7. The method of claim 5 wherein the single-chain T cell receptor polypeptide is a TCR a chain.

8. The method of claim 5 wherein the single-chain T cell receptor polypeptide is a TCR b chain.

9. The soluble single-chain T cell receptor conjugate complex of claim 1-4 further comprising a plurality of biologically active molecules, wherein the biologically active molecules are selected from the group consisting of cytotoxic molecules, toxins, chemotherapeutic agents, anti-cancer drugs, detectable labels, fluorescent compounds, electron transfer agents, enzymes, and radioactive compounds, wherein the biologically active molecules are covalently linked to the soluble single-chain T cell receptor conjugate complex, wherein the resulting conjugate is soluble.

10. A therapeutic composition for treatment of disorders comprising a therapeutically effective amount of the single-chain T cell receptor fusion complex of any one of claims 1-4 and 9 and a sterile, pharmaceutically acceptable carrier vehicle.

11. The therapeutic composition of claim 10 wherein the soluble single-chain T cell receptor fusion complex is directed to treatment of a malignant disorder.

12. The therapeutic composition of claim 10 wherein the soluble single-chain T cell receptor fusion complex is directed to treatment of an autoimmune disorder.

13. The therapeutic composition of claim 10 wherein the soluble single-chain T cell receptor fusion complex is directed to treatment of an inflammatory response.

14. The therapeutic composition of claim 10 wherein the soluble single-chain T cell receptor fusion complex is directed to treatment of an infectious disease.

15. The therapeutic composition of claim 10 wherein the soluble single-chain T cell receptor fusion complex is directed to treatment of a viral infection.

16. A diagnostic composition for indication of disorders comprising a diagnostically effective amount of the soluble single-chain T cell receptor conjugate complex of claim 9 and a sterile, pharmaceutically acceptable carrier vehicle.

17. The diagnostic composition of claim 16 wherein the soluble single-chain T cell receptor conjugate complex is a detectably labeled molecule suitable for diagnostic or imaging studies.

18. A nucleic acid sequence encoding a soluble single-chain T cell receptor fusion complex comprising a single-chain T cell receptor polypeptide and an IL-2 polypeptide connected by a peptide linker.

19. The nucleic acid sequence of claim 18 wherein the single-chain T cell receptor polypeptide is specific for recognition of a particular antigen.

20. The nucleic acid sequence of claim 18 wherein the single-chain T cell receptor polypeptide is a heterodimer comprising a and b chain TCR.

21. The nucleic acid sequence of claim 18-20 wherein the single-chain T cell receptor polypeptide a and b chains are linked through a non-covalent linkage.

22. The nucleic acid sequence of any one of claims 18 to 21 wherein the nucleic acid is DNA.

23. The nucleic acid sequence of any one of claims 18 to 21 wherein the nucleic acid is RNA.

## Patentansprüche

1. Löslicher Einzelketten-T-Zell-Rezeptor-Fusionskomplex, umfassend ein Einzelketten-T-Zell-Rezeptor-Polypeptid und ein Interleukin-2 (IL-2)-Polypeptid, welche durch einen Peptidlinker verbunden sind.

2. Löslicher Einzelketten-T-Zell-Rezeptor-Fusionskomplex gemäß Anspruch 1, wobei das Einzelketten-T-Zell-Rezeptor-Polypeptid für die Erkennung eines bestimmten Antigens spezifisch ist.

3. Löslicher Einzelketten-T-Zell-Rezeptor-Fusionskomplex, gemäß Anspruch 1 oder 2, wobei das Einzelketten-T-Zell-Rezeptor-Polypeptid ein Heterodimer ist, das einen a- und b-Ketten-TCR umfasst.

4. Löslicher Einzelketten-T-Zell-Rezeptor-Fusionskomplex gemäß Anspruch 1-3, wobei die Einzelketten-T-Zell-Rezeptor-Polypeptid-a- und -b-Ketten durch eine nicht-kovalente Verknüpfung verknüpft sind.

5. Verfahren zur Herstellung eines löslichen Einzelketten-T-Zell-Rezeptor-Fusionskomplexes, wobei das Verfahren umfasst:
Bereitstellen einer Einzelketten-T-Zell-Rezeptor-Polypeptid-Kette oder eines Subfragmentes davon;
Bereitstellen eines IL-2-Polypeptids;
Verbinden der Einzelketten-T-Zell-Rezeptor-Kette und des IL-2-Polypeptids mit einem Peptidlinker und
Gewinnung des verknüpften Einzelketten-T-Zell-Rezeptor-Fusions-Polypeptid-Komplexes, wodurch ein löslicher Einzelketten-T-Zell-Rezeptor-Fusionskomplex hergestellt wird.

6. Verfahren gemäß Anspruch 5, wobei das Einzelketten-T-Zell-Rezeptor-Polypeptid für eine Erkennung eines bestimmten Antigens spezifisch ist.

7. Verfahren gemäß Anspruch 5, wobei das Einzelketten-T-Zell-Rezeptor-Polypeptid eine TCR-a-Kette ist.

8. Verfahren gemäß Anspruch 5, wobei das Einzelketten-T-Zell-Rezeptor-Polypeptid eine TCR-b-Kette ist.

9. Löslicher Einzelketten-T-Zell-Rezeptor-Konjugat-Komplex gemäß Anspruch 1-4, des Weiteren umfassend eine Vielzahl biologisch aktiver Moleküle, wobei die biologisch aktiven Moleküle ausgewählt sind aus der Gruppe, bestehend aus zytotoxischen Molekülen, Toxinen, Chemotherapeutika, Antikrebsarzneimitteln, detektierbaren Markierungen, fluoreszierenden Verbindungen, Elektronenübertragungsmitteln, Enzymen und radioaktiven Verbindungen, wobei die biologisch aktiven Moleküle kovalent an den löslichen Einzelketten-T-Zell-Rezeptor-Konjugat-Komplex gebunden sind, wobei das resultierende Konjugat löslich ist.

10. Therapeutische Zusammensetzung zur Behandlung von Störungen, umfassend eine therapeutisch wirksame Menge des Einzelketten-T-Zell-Rezeptor-Fusionskomplexes gemäß einem der Ansprüche 1-4 und 9 und ein steriles pharmazeutisch verträgliches Trägervehikel.

11. Therapeutische Zusammensetzung gemäß Anspruch 10, wobei der lösliche Einzelketten-T-Zell-Rezeptor-Fusionskomplex auf die Behandlung einer malignen Störung gerichtet ist.

12. Therapeutische Zusammensetzung gemäß Anspruch 10, wobei der lösliche Einzelketten-T-Zell-Rezeptor-Fusionskomplex auf die Behandlung einer Autoimmunstörung gerichtet ist.

13. Therapeutische Zusammensetzung gemäß Anspruch 10, wobei der lösliche Einzelketten-T-Zell-Rezeptor-Fusionskomplex auf die Behandlung einer inflammatorischen Reaktion gerichtet ist.

14. Therapeutische Zusammensetzung gemäß Anspruch 10, wobei der lösliche Einzelketten-T-Zell-Rezeptor-Fusionskomplex auf die Behandlung einer infektiösen Erkrankung gerichtet ist.

15. Therapeutische Zusammensetzung gemäß Anspruch 10, wobei der lösliche Einzelketten-T-Zell-Rezeptor-Fusionskomplex auf die Behandlung einer Virusinfektion gerichtet ist.

16. Diagnostische Zusammensetzung zur Indikation von Störungen, umfassend eine diagnostisch wirksame Menge des löslichen Einzelketten-T-Zell-Rezeptor-Konjugat-Komplexes gemäß Anspruch 9 und ein steriles pharmazeutisch verträgliches Trägervehikel.

17. Diagnostische Zusammensetzung gemäß Anspruch 16, wobei der lösliche Einzelketten-T-Zell-Rezeptor-Konjugat-Komplex ein detektierbar markiertes Molekül ist, das für diagnostische oder bildgebende Studien geeignet ist.

18. Nucleinsäuresequenz, die einen löslichen Einzelketten-T-Zell-Rezeptor-Fusionskomplex, umfassend ein Einzelketten-T-Zell-Rezeptor-Polypeptid und ein IL-2-Polypeptid, welche durch einen Peptidlinker verbunden sind, codiert.

19. Nucleinsäuresequenz gemäß Anspruch 18, wobei das Einzelketten-T-Zell-Rezeptor-Polypeptid für die Erkennung eines bestimmten Antigens spezifisch ist.

20. Nucleinsäuresequenz gemäß Anspruch 18, wobei das Einzelketten-T-Zell-Rezeptor-Polypeptid ein Heterodimer ist, das a- und b-Ketten-TCR umfasst.

21. Nucleinsäuresequenz gemäß Anspruch 18-20, wobei die Einzelketten-T-Zell-Rezeptor-Polypeptid-a- und -b-Ketten durch eine nicht-kovalente Verknüpfung verknüpft sind.

22. Nucleinsäuresequenz gemäß einem der Ansprüche 18 bis 21, wobei die Nucleinsäure DNA ist.

23. Nucleinsäuresequenz gemäß einem der Ansprüche 18 bis 21, wobei die Nucleinsäure RNA ist.

## Revendications

1. Complexe de fusion à récepteur de lymphocyte T monocaténaire soluble comprenant un polypeptide monocaténaire de récepteur de lymphocyte T et un polypeptide d'interleukine-2 (IL-2) connectés par un lieur peptidique.

2. Complexe de fusion à récepteur de lymphocyte T monocaténaire soluble selon la revendication 1 dans lequel le polypeptide monocaténaire de récepteur de lymphocyte T est spécifique pour la reconnaissance d'un antigène particulier.

3. Complexe de fusion à récepteur de lymphocyte T monocaténaire soluble selon la revendication 1 ou 2 dans lequel le polypeptide monocaténaire de récepteur de lymphocyte T est un hétérodimère comprenant un TCR à chaînes a et b.

4. Complexe de fusion à récepteur de lymphocyte T monocaténaire soluble selon les revendications 1 à 3 dans lequel les chaînes a et b du polypeptide monocaténaire de récepteur de lymphocyte T sont liées par l'intermédiaire d'une liaison non covalente.

5. Procédé de préparation d'un complexe de fusion à récepteur de lymphocyte T monocaténaire soluble, le procédé comprenant :
la fourniture d'une chaîne polypeptidique monocaténaire de récepteur de lymphocyte T, ou d'un sous-fragment de celle-ci ;
la fourniture d'un polypeptide d'IL-2 ;
la connexion de la chaîne monocaténaire de récepteur de lymphocyte T et du polypeptide d'IL-2 à un lieur peptidique ; et
la récupération du complexe polypeptidique de fusion à récepteur de lymphocyte T monocaténaire lié, pour ainsi générer un complexe de fusion à récepteur de lymphocyte T monocaténaire soluble.

6. Procédé selon la revendication 5 dans lequel le polypeptide monocaténaire de récepteur de lymphocyte T est spécifique pour la reconnaissance d'un antigène particulier.

7. Procédé selon la revendication 5 dans lequel le polypeptide monocaténaire de récepteur de lymphocyte T est une chaîne a de TCR.

8. Procédé selon la revendication 5 dans lequel le polypeptide monocaténaire de récepteur de lymphocyte T est une chaîne b de TCR.

9. Complexe de conjugué à récepteur de lymphocyte T monocaténaire soluble selon les revendications 1 à 4 comprenant en outre une pluralité de molécules biologiquement actives, dans lequel les molécules biologiquement actives sont sélectionnées dans le groupe constitué des molécules cytotoxiques, des toxines, des agents chimiothérapeutiques, des médicaments anticancéreux, des marqueurs détectables, des composés fluorescents, des agents de transfert d'électrons, des enzymes, et des composés radioactifs, dans lequel les molécules biologiquement actives sont liées par liaison covalente au complexe de conjugué à récepteur de lymphocyte T monocaténaire soluble, dans lequel le conjugué résultant est soluble.

10. Composition thérapeutique pour le traitement des troubles comprenant une quantité thérapeutiquement efficace du complexe de fusion à récepteur de lymphocyte T monocaténaire selon l'une quelconque des revendications 1 à 4 et 9 et un véhicule de support stérile pharmaceutiquement acceptable.

11. Composition thérapeutique selon la revendication 10 dans laquelle le complexe de fusion à récepteur de lymphocyte T monocaténaire soluble est dirigé vers le traitement d'un trouble malin.

12. Composition thérapeutique selon la revendication 10 dans laquelle le complexe de fusion à récepteur de lymphocyte T monocaténaire soluble est dirigé vers le traitement d'un trouble auto-immun.

13. Composition thérapeutique selon la revendication 10 dans laquelle le complexe de fusion à récepteur de lymphocyte T monocaténaire soluble est dirigé vers le traitement d'une réponse inflammatoire.

14. Composition thérapeutique selon la revendication 10 dans laquelle le complexe de fusion à récepteur de lymphocyte T monocaténaire soluble est dirigé vers le traitement d'une maladie infectieuse.

15. Composition thérapeutique selon la revendication 10 dans laquelle le complexe de fusion à récepteur de lymphocyte T monocaténaire soluble est dirigé vers le traitement d'une infection virale.

16. Composition diagnostique pour l'indication de troubles comprenant une quantité efficace d'un point de vue diagnostique du complexe de conjugué à récepteur de lymphocyte T monocaténaire soluble selon la revendication 9 et un véhicule de support stérile pharmaceutiquement acceptable.

17. Composition diagnostique selon la revendication 16 dans laquelle le complexe de conjugué à récepteur de lymphocyte T monocaténaire soluble est une molécule à marquage détectable appropriée pour des études de diagnostic ou d'imagerie.

18. Séquence d'acide nucléique codant pour un complexe de fusion à récepteur de lymphocyte T monocaténaire soluble comprenant un polypeptide monocaténaire de récepteur de lymphocyte T et un polypeptide d'IL-2 connectés par un lieur peptidique.

19. Séquence d'acide nucléique selon la revendication 18 dans laquelle le polypeptide monocaténaire de récepteur de lymphocyte T est spécifique pour la reconnaissance d'un antigène particulier.

20. Séquence d'acide nucléique selon la revendication 18 dans laquelle le polypeptide monocaténaire de récepteur de lymphocyte T est un hétérodimère comprenant un TCR à chaînes a et b.

21. Séquence d'acide nucléique selon les revendications 18 à 20 dans lequel les chaînes a et b du polypeptide monocaténaire de récepteur de lymphocyte T sont liées par l'intermédiaire d'une liaison non covalente.

22. Séquence d'acide nucléique selon l'une quelconque des revendications 18 à 21 dans laquelle l'acide nucléique est un ADN.

23. Séquence d'acide nucléique selon l'une quelconque des revendications 18 à 21 dans laquelle l'acide nucléique est un ARN.
